# EUROPEAN PATENT APPLICATION

(11) **EP 3 666 281 A1**
(43) Date of publication of application: **17.06.2020**
(21) Application number: 18212797.7
(22) Date of filing: 14.12.2018
(51) Int. Cl.: A61K 38/17, C07K 14/705, A61P 3/10

(54) **COMPOSITIONS COMPRISING SORTILIN-1**

(71) Applicant: Insusense ApS, 2100 Copenhagen (DK)
(72) Inventor: Kjølby, Mads Fuglsang, DK-2100 Copenhagen (DK); Nykjær, Anders, DK-8240 Risskov (DK); Thirup, Søren, Aarhus C (DK)
(74) Representative: Gill Jennings & Every LLP

(57) **Abstract**

The invention provides composition comprising a fragment, derivative or variant of sortilin, or a sortilin mimetic, for use in medicine, wherein the sortilin mimetic is not SorCS1, SorCS2, SorCS3 or SorLA. The composition preferably for use in treating or preventing diabetes mellitus, insulin resistance, obesity, metabolic disorders, polycystic ovary syndrome, non-alcoholic fatty liver disease, retinopathy, neuropathy, nephropathy, Alzheimer's disease and/or cardiovascular disease in a subject in need thereof. Also provided are isolated polypeptides, nucleic acids encoding the polypeptides, vectors, host cells and expression systems containing the nucleic acids and methods for making the polypeptides.

SEQUENCE LISTING

<110>  Insusense ApS

<120>  Compositions

<130>  RMC00128EP

<160>  27

<170>  PatentIn version 3.5

<210>  1
<211>  540
<212>  PRT
<213>  Artificial Sequence

<220>
<223>  VPS10p Domain- isoform 1

<400>  1

Arg Gly Gly Arg Trp Arg Arg Ser Ala Pro Gly Glu Asp Glu Glu Cys
1               5                   10                  15

Gly Arg Val Arg Asp Phe Val Ala Lys Leu Ala Asn Asn Thr His Gln
            20                  25                  30

His Val Phe Asp Asp Leu Arg Gly Ser Val Ser Leu Ser Trp Val Gly
        35                  40                  45

Asp Ser Thr Gly Val Ile Leu Val Leu Thr Thr Phe His Val Pro Leu
    50                  55                  60

Val Ile Met Thr Phe Gly Gln Ser Lys Leu Tyr Arg Ser Glu Asp Tyr
65                  70                  75                  80

Gly Lys Asn Phe Lys Asp Ile Thr Asp Leu Ile Asn Asn Thr Phe Ile
                85                  90                  95

Arg Thr Glu Phe Gly Met Ala Ile Gly Pro Glu Asn Ser Gly Lys Val
            100                 105                 110

Val Leu Thr Ala Glu Val Ser Gly Gly Ser Arg Gly Gly Arg Ile Phe
        115                 120                 125

Arg Ser Ser Asp Phe Ala Lys Asn Phe Val Gln Thr Asp Leu Pro Phe
    130                 135                 140

His Pro Leu Thr Gln Met Met Tyr Ser Pro Gln Asn Ser Asp Tyr Leu
145                 150                 155                 160

Leu Ala Leu Ser Thr Glu Asn Gly Leu Trp Val Ser Lys Asn Phe Gly

165                 170                 175

Gly Lys Trp Glu Glu Ile His Lys Ala Val Cys Leu Ala Lys Trp Gly
            180                 185                 190

Ser Asp Asn Thr Ile Phe Phe Thr Thr Tyr Ala Asn Gly Ser Cys Lys
        195                 200                 205

Ala Asp Leu Gly Ala Leu Glu Leu Trp Arg Thr Ser Asp Leu Gly Lys
    210                 215                 220

Ser Phe Lys Thr Ile Gly Val Lys Ile Tyr Ser Phe Gly Leu Gly Gly
225                 230                 235                 240

Arg Phe Leu Phe Ala Ser Val Met Ala Asp Lys Asp Thr Thr Arg Arg
                245                 250                 255

Ile His Val Ser Thr Asp Gln Gly Asp Thr Trp Ser Met Ala Gln Leu
            260                 265                 270

Pro Ser Val Gly Gln Glu Gln Phe Tyr Ser Ile Leu Ala Ala Asn Asp
        275                 280                 285

Asp Met Val Phe Met His Val Asp Glu Pro Gly Asp Thr Gly Phe Gly
    290                 295                 300

Thr Ile Phe Thr Ser Asp Asp Arg Gly Ile Val Tyr Ser Lys Ser Leu
305                 310                 315                 320

Asp Arg His Leu Tyr Thr Thr Thr Gly Gly Glu Thr Asp Phe Thr Asn
                325                 330                 335

Val Thr Ser Leu Arg Gly Val Tyr Ile Thr Ser Val Leu Ser Glu Asp
            340                 345                 350

Asn Ser Ile Gln Thr Met Ile Thr Phe Asp Gln Gly Gly Arg Trp Thr
        355                 360                 365

His Leu Arg Lys Pro Glu Asn Ser Glu Cys Asp Ala Thr Ala Lys Asn
    370                 375                 380

Lys Asn Glu Cys Ser Leu His Ile His Ala Ser Tyr Ser Ile Ser Gln
385                 390                 395                 400

Lys Leu Asn Val Pro Met Ala Pro Leu Ser Glu Pro Asn Ala Val Gly
                405                 410                 415

Ile Val Ile Ala His Gly Ser Val Gly Asp Ala Ile Ser Val Met Val
            420                 425                 430

Pro Asp Val Tyr Ile Ser Asp Asp Gly Gly Tyr Ser Trp Thr Lys Met
        435                 440                 445

Leu Glu Gly Pro His Tyr Tyr Thr Ile Leu Asp Ser Gly Gly Ile Ile
    450                 455                 460

Val Ala Ile Glu His Ser Ser Arg Pro Ile Asn Val Ile Lys Phe Ser
465                 470                 475                 480

Thr Asp Glu Gly Gln Cys Trp Gln Thr Tyr Thr Phe Thr Arg Asp Pro
                485                 490                 495

Ile Tyr Phe Thr Gly Leu Ala Ser Glu Pro Gly Ala Arg Ser Met Asn
            500                 505                 510

Ile Ser Ile Trp Gly Phe Thr Glu Ser Phe Leu Thr Ser Gln Trp Val
        515                 520                 525

Ser Tyr Thr Ile Asp Phe Lys Asp Ile Leu Glu Arg
    530                 535                 540

<210>  2
<211>  597
<212>  PRT
<213>  Artificial Sequence

<220>
<223>  VPS10p Domain + 10CCa Domain- isoform 1

<400>  2

Arg Gly Gly Arg Trp Arg Arg Ser Ala Pro Gly Glu Asp Glu Glu Cys
1               5                   10                  15

Gly Arg Val Arg Asp Phe Val Ala Lys Leu Ala Asn Asn Thr His Gln
            20                  25                  30

His Val Phe Asp Asp Leu Arg Gly Ser Val Ser Leu Ser Trp Val Gly
        35                  40                  45

Asp Ser Thr Gly Val Ile Leu Val Leu Thr Thr Phe His Val Pro Leu
    50                  55                  60

Val Ile Met Thr Phe Gly Gln Ser Lys Leu Tyr Arg Ser Glu Asp Tyr
65                  70                  75                  80

Gly Lys Asn Phe Lys Asp Ile Thr Asp Leu Ile Asn Asn Thr Phe Ile

85                  90                  95

Arg Thr Glu Phe Gly Met Ala Ile Gly Pro Glu Asn Ser Gly Lys Val
            100                 105                 110

Val Leu Thr Ala Glu Val Ser Gly Gly Ser Arg Gly Gly Arg Ile Phe
        115                 120                 125

Arg Ser Ser Asp Phe Ala Lys Asn Phe Val Gln Thr Asp Leu Pro Phe
    130                 135                 140

His Pro Leu Thr Gln Met Met Tyr Ser Pro Gln Asn Ser Asp Tyr Leu
145                 150                 155                 160

Leu Ala Leu Ser Thr Glu Asn Gly Leu Trp Val Ser Lys Asn Phe Gly
                165                 170                 175

Gly Lys Trp Glu Glu Ile His Lys Ala Val Cys Leu Ala Lys Trp Gly
            180                 185                 190

Ser Asp Asn Thr Ile Phe Phe Thr Thr Tyr Ala Asn Gly Ser Cys Lys
        195                 200                 205

Ala Asp Leu Gly Ala Leu Glu Leu Trp Arg Thr Ser Asp Leu Gly Lys
    210                 215                 220

Ser Phe Lys Thr Ile Gly Val Lys Ile Tyr Ser Phe Gly Leu Gly Gly
225                 230                 235                 240

Arg Phe Leu Phe Ala Ser Val Met Ala Asp Lys Asp Thr Thr Arg Arg
                245                 250                 255

Ile His Val Ser Thr Asp Gln Gly Asp Thr Trp Ser Met Ala Gln Leu
            260                 265                 270

Pro Ser Val Gly Gln Glu Gln Phe Tyr Ser Ile Leu Ala Ala Asn Asp
        275                 280                 285

Asp Met Val Phe Met His Val Asp Glu Pro Gly Asp Thr Gly Phe Gly
    290                 295                 300

Thr Ile Phe Thr Ser Asp Asp Arg Gly Ile Val Tyr Ser Lys Ser Leu
305                 310                 315                 320

Asp Arg His Leu Tyr Thr Thr Thr Gly Gly Glu Thr Asp Phe Thr Asn
                325                 330                 335

Val Thr Ser Leu Arg Gly Val Tyr Ile Thr Ser Val Leu Ser Glu Asp
        340                 345                 350

Asn Ser Ile Gln Thr Met Ile Thr Phe Asp Gln Gly Gly Arg Trp Thr
    355                 360                 365

His Leu Arg Lys Pro Glu Asn Ser Glu Cys Asp Ala Thr Ala Lys Asn
    370                 375                 380

Lys Asn Glu Cys Ser Leu His Ile His Ala Ser Tyr Ser Ile Ser Gln
385                 390                 395                 400

Lys Leu Asn Val Pro Met Ala Pro Leu Ser Glu Pro Asn Ala Val Gly
                405                 410                 415

Ile Val Ile Ala His Gly Ser Val Gly Asp Ala Ile Ser Val Met Val
            420                 425                 430

Pro Asp Val Tyr Ile Ser Asp Asp Gly Gly Tyr Ser Trp Thr Lys Met
        435                 440                 445

Leu Glu Gly Pro His Tyr Tyr Thr Ile Leu Asp Ser Gly Gly Ile Ile
    450                 455                 460

Val Ala Ile Glu His Ser Ser Arg Pro Ile Asn Val Ile Lys Phe Ser
465                 470                 475                 480

Thr Asp Glu Gly Gln Cys Trp Gln Thr Tyr Thr Phe Thr Arg Asp Pro
                485                 490                 495

Ile Tyr Phe Thr Gly Leu Ala Ser Glu Pro Gly Ala Arg Ser Met Asn
            500                 505                 510

Ile Ser Ile Trp Gly Phe Thr Glu Ser Phe Leu Thr Ser Gln Trp Val
        515                 520                 525

Ser Tyr Thr Ile Asp Phe Lys Asp Ile Leu Glu Arg Asn Cys Glu Glu
    530                 535                 540

Lys Asp Tyr Thr Ile Trp Leu Ala His Ser Thr Asp Pro Glu Asp Tyr
545                 550                 555                 560

Glu Asp Gly Cys Ile Leu Gly Tyr Lys Glu Gln Phe Leu Arg Leu Arg
                565                 570                 575

Lys Ser Ser Met Cys Gln Asn Gly Arg Asp Tyr Val Val Thr Lys Gln
            580                 585                 590

Pro Ser Ile Cys Leu
        595

<210>  3
<211>  623
<212>  PRT
<213>  Artificial Sequence

<220>
<223>  VPS10p Domain + 10CCb Domain- isoform 1

<400>  3

Arg Gly Gly Arg Trp Arg Arg Ser Ala Pro Gly Glu Asp Glu Glu Cys
1               5                   10                  15

Gly Arg Val Arg Asp Phe Val Ala Lys Leu Ala Asn Asn Thr His Gln
            20                  25                  30

His Val Phe Asp Asp Leu Arg Gly Ser Val Ser Leu Ser Trp Val Gly
        35                  40                  45

Asp Ser Thr Gly Val Ile Leu Val Leu Thr Thr Phe His Val Pro Leu
    50                  55                  60

Val Ile Met Thr Phe Gly Gln Ser Lys Leu Tyr Arg Ser Glu Asp Tyr
65                  70                  75                  80

Gly Lys Asn Phe Lys Asp Ile Thr Asp Leu Ile Asn Asn Thr Phe Ile
                85                  90                  95

Arg Thr Glu Phe Gly Met Ala Ile Gly Pro Glu Asn Ser Gly Lys Val
            100                 105                 110

Val Leu Thr Ala Glu Val Ser Gly Gly Ser Arg Gly Gly Arg Ile Phe
        115                 120                 125

Arg Ser Ser Asp Phe Ala Lys Asn Phe Val Gln Thr Asp Leu Pro Phe
    130                 135                 140

His Pro Leu Thr Gln Met Met Tyr Ser Pro Gln Asn Ser Asp Tyr Leu
145                 150                 155                 160

Leu Ala Leu Ser Thr Glu Asn Gly Leu Trp Val Ser Lys Asn Phe Gly
                165                 170                 175

Gly Lys Trp Glu Glu Ile His Lys Ala Val Cys Leu Ala Lys Trp Gly
            180                 185                 190

Ser Asp Asn Thr Ile Phe Phe Thr Thr Tyr Ala Asn Gly Ser Cys Lys
        195                 200                 205

Ala Asp Leu Gly Ala Leu Glu Leu Trp Arg Thr Ser Asp Leu Gly Lys
    210                 215                 220

Ser Phe Lys Thr Ile Gly Val Lys Ile Tyr Ser Phe Gly Leu Gly Gly
225                 230                 235                 240

Arg Phe Leu Phe Ala Ser Val Met Ala Asp Lys Asp Thr Thr Arg Arg
                245                 250                 255

Ile His Val Ser Thr Asp Gln Gly Asp Thr Trp Ser Met Ala Gln Leu
            260                 265                 270

Pro Ser Val Gly Gln Glu Gln Phe Tyr Ser Ile Leu Ala Ala Asn Asp
        275                 280                 285

Asp Met Val Phe Met His Val Asp Glu Pro Gly Asp Thr Gly Phe Gly
    290                 295                 300

Thr Ile Phe Thr Ser Asp Asp Arg Gly Ile Val Tyr Ser Lys Ser Leu
305                 310                 315                 320

Asp Arg His Leu Tyr Thr Thr Thr Gly Gly Glu Thr Asp Phe Thr Asn
                325                 330                 335

Val Thr Ser Leu Arg Gly Val Tyr Ile Thr Ser Val Leu Ser Glu Asp
            340                 345                 350

Asn Ser Ile Gln Thr Met Ile Thr Phe Asp Gln Gly Gly Arg Trp Thr
        355                 360                 365

His Leu Arg Lys Pro Glu Asn Ser Glu Cys Asp Ala Thr Ala Lys Asn
    370                 375                 380

Lys Asn Glu Cys Ser Leu His Ile His Ala Ser Tyr Ser Ile Ser Gln
385                 390                 395                 400

Lys Leu Asn Val Pro Met Ala Pro Leu Ser Glu Pro Asn Ala Val Gly
                405                 410                 415

Ile Val Ile Ala His Gly Ser Val Gly Asp Ala Ile Ser Val Met Val
            420                 425                 430

Pro Asp Val Tyr Ile Ser Asp Asp Gly Gly Tyr Ser Trp Thr Lys Met
        435                 440                 445

Leu Glu Gly Pro His Tyr Tyr Thr Ile Leu Asp Ser Gly Gly Ile Ile
    450                 455                 460

Val Ala Ile Glu His Ser Ser Arg Pro Ile Asn Val Ile Lys Phe Ser
465                 470                 475                 480

Thr Asp Glu Gly Gln Cys Trp Gln Thr Tyr Thr Phe Thr Arg Asp Pro
                485                 490                 495

Ile Tyr Phe Thr Gly Leu Ala Ser Glu Pro Gly Ala Arg Ser Met Asn
            500                 505                 510

Ile Ser Ile Trp Gly Phe Thr Glu Ser Phe Leu Thr Ser Gln Trp Val
        515                 520                 525

Ser Tyr Thr Ile Asp Phe Lys Asp Ile Leu Glu Arg Cys Ser Leu Glu
    530                 535                 540

Asp Phe Leu Cys Asp Phe Gly Tyr Tyr Arg Pro Glu Asn Asp Ser Lys
545                 550                 555                 560

Cys Val Glu Gln Pro Glu Leu Lys Gly His Asp Leu Glu Phe Cys Leu
                565                 570                 575

Tyr Gly Arg Glu Glu His Leu Thr Thr Asn Gly Tyr Arg Lys Ile Pro
            580                 585                 590

Gly Asp Lys Cys Gln Gly Gly Val Asn Pro Val Arg Glu Val Lys Asp
        595                 600                 605

Leu Lys Lys Lys Cys Thr Ser Asn Phe Leu Ser Pro Glu Lys Gln
    610                 615                 620

<210>  4
<211>  680
<212>  PRT
<213>  Artificial Sequence

<220>
<223>  VPS10p Domain + 10CCa Domain + 10CCb Domain- isoform 1

<400>  4

Arg Gly Gly Arg Trp Arg Arg Ser Ala Pro Gly Glu Asp Glu Glu Cys
1               5                   10                  15

Gly Arg Val Arg Asp Phe Val Ala Lys Leu Ala Asn Asn Thr His Gln
            20                  25                  30

His Val Phe Asp Asp Leu Arg Gly Ser Val Ser Leu Ser Trp Val Gly
        35                  40                  45

Asp Ser Thr Gly Val Ile Leu Val Leu Thr Thr Phe His Val Pro Leu
    50                  55                  60

Val Ile Met Thr Phe Gly Gln Ser Lys Leu Tyr Arg Ser Glu Asp Tyr
65                  70                  75                  80

Gly Lys Asn Phe Lys Asp Ile Thr Asp Leu Ile Asn Asn Thr Phe Ile
                85                  90                  95

Arg Thr Glu Phe Gly Met Ala Ile Gly Pro Glu Asn Ser Gly Lys Val
            100                 105                 110

Val Leu Thr Ala Glu Val Ser Gly Gly Ser Arg Gly Gly Arg Ile Phe
        115                 120                 125

Arg Ser Ser Asp Phe Ala Lys Asn Phe Val Gln Thr Asp Leu Pro Phe
    130                 135                 140

His Pro Leu Thr Gln Met Met Tyr Ser Pro Gln Asn Ser Asp Tyr Leu
145                 150                 155                 160

Leu Ala Leu Ser Thr Glu Asn Gly Leu Trp Val Ser Lys Asn Phe Gly
                165                 170                 175

Gly Lys Trp Glu Glu Ile His Lys Ala Val Cys Leu Ala Lys Trp Gly
            180                 185                 190

Ser Asp Asn Thr Ile Phe Phe Thr Thr Tyr Ala Asn Gly Ser Cys Lys
        195                 200                 205

Ala Asp Leu Gly Ala Leu Glu Leu Trp Arg Thr Ser Asp Leu Gly Lys
    210                 215                 220

Ser Phe Lys Thr Ile Gly Val Lys Ile Tyr Ser Phe Gly Leu Gly Gly
225                 230                 235                 240

Arg Phe Leu Phe Ala Ser Val Met Ala Asp Lys Asp Thr Thr Arg Arg
                245                 250                 255

Ile His Val Ser Thr Asp Gln Gly Asp Thr Trp Ser Met Ala Gln Leu
            260                 265                 270

Pro Ser Val Gly Gln Glu Gln Phe Tyr Ser Ile Leu Ala Ala Asn Asp
        275                 280                 285

Asp Met Val Phe Met His Val Asp Glu Pro Gly Asp Thr Gly Phe Gly
    290                 295                 300

Thr Ile Phe Thr Ser Asp Asp Arg Gly Ile Val Tyr Ser Lys Ser Leu
305                 310                 315                 320

Asp Arg His Leu Tyr Thr Thr Thr Gly Gly Glu Thr Asp Phe Thr Asn
                325                 330                 335

Val Thr Ser Leu Arg Gly Val Tyr Ile Thr Ser Val Leu Ser Glu Asp
            340                 345                 350

Asn Ser Ile Gln Thr Met Ile Thr Phe Asp Gln Gly Gly Arg Trp Thr
        355                 360                 365

His Leu Arg Lys Pro Glu Asn Ser Glu Cys Asp Ala Thr Ala Lys Asn
    370                 375                 380

Lys Asn Glu Cys Ser Leu His Ile His Ala Ser Tyr Ser Ile Ser Gln
385                 390                 395                 400

Lys Leu Asn Val Pro Met Ala Pro Leu Ser Glu Pro Asn Ala Val Gly
                405                 410                 415

Ile Val Ile Ala His Gly Ser Val Gly Asp Ala Ile Ser Val Met Val
            420                 425                 430

Pro Asp Val Tyr Ile Ser Asp Asp Gly Gly Tyr Ser Trp Thr Lys Met
        435                 440                 445

Leu Glu Gly Pro His Tyr Tyr Thr Ile Leu Asp Ser Gly Gly Ile Ile
    450                 455                 460

Val Ala Ile Glu His Ser Ser Arg Pro Ile Asn Val Ile Lys Phe Ser
465                 470                 475                 480

Thr Asp Glu Gly Gln Cys Trp Gln Thr Tyr Thr Phe Thr Arg Asp Pro
                485                 490                 495

Ile Tyr Phe Thr Gly Leu Ala Ser Glu Pro Gly Ala Arg Ser Met Asn
            500                 505                 510

Ile Ser Ile Trp Gly Phe Thr Glu Ser Phe Leu Thr Ser Gln Trp Val
        515                 520                 525

Ser Tyr Thr Ile Asp Phe Lys Asp Ile Leu Glu Arg Asn Cys Glu Glu
    530                 535                 540

Lys Asp Tyr Thr Ile Trp Leu Ala His Ser Thr Asp Pro Glu Asp Tyr
545                 550                 555                 560

Glu Asp Gly Cys Ile Leu Gly Tyr Lys Glu Gln Phe Leu Arg Leu Arg
                565                 570                 575

Lys Ser Ser Met Cys Gln Asn Gly Arg Asp Tyr Val Val Thr Lys Gln
            580                 585                 590

Pro Ser Ile Cys Leu Cys Ser Leu Glu Asp Phe Leu Cys Asp Phe Gly
        595                 600                 605

Tyr Tyr Arg Pro Glu Asn Asp Ser Lys Cys Val Glu Gln Pro Glu Leu
    610                 615                 620

Lys Gly His Asp Leu Glu Phe Cys Leu Tyr Gly Arg Glu Glu His Leu
625                 630                 635                 640

Thr Thr Asn Gly Tyr Arg Lys Ile Pro Gly Asp Lys Cys Gln Gly Gly
                645                 650                 655

Val Asn Pro Val Arg Glu Val Lys Asp Leu Lys Lys Lys Cys Thr Ser
            660                 665                 670

Asn Phe Leu Ser Pro Glu Lys Gln
        675                 680

<210>  5
<211>  57
<212>  PRT
<213>  Artificial Sequence

<220>
<223>  10CCa Domain- isoform 1

<400>  5

Asn Cys Glu Glu Lys Asp Tyr Thr Ile Trp Leu Ala His Ser Thr Asp
1               5                   10                  15

Pro Glu Asp Tyr Glu Asp Gly Cys Ile Leu Gly Tyr Lys Glu Gln Phe
            20                  25                  30

Leu Arg Leu Arg Lys Ser Ser Met Cys Gln Asn Gly Arg Asp Tyr Val
        35                  40                  45

Val Thr Lys Gln Pro Ser Ile Cys Leu
    50                  55

<210>  6
<211>  83
<212>  PRT
<213>  Artificial Sequence

<220>
<223>  10CCb Domain- isoform 1

<400>  6

Cys Ser Leu Glu Asp Phe Leu Cys Asp Phe Gly Tyr Tyr Arg Pro Glu
1               5                   10                  15

Asn Asp Ser Lys Cys Val Glu Gln Pro Glu Leu Lys Gly His Asp Leu
            20                  25                  30

Glu Phe Cys Leu Tyr Gly Arg Glu Glu His Leu Thr Thr Asn Gly Tyr
        35                  40                  45

Arg Lys Ile Pro Gly Asp Lys Cys Gln Gly Gly Val Asn Pro Val Arg
    50                  55                  60

Glu Val Lys Asp Leu Lys Lys Lys Cys Thr Ser Asn Phe Leu Ser Pro
65                  70                  75                  80

Glu Lys Gln



<210>  7
<211>  140
<212>  PRT
<213>  Artificial Sequence

<220>
<223>  10CCa + 10CCb Domain- isoform 1

<400>  7

Asn Cys Glu Glu Lys Asp Tyr Thr Ile Trp Leu Ala His Ser Thr Asp
1               5                   10                  15

Pro Glu Asp Tyr Glu Asp Gly Cys Ile Leu Gly Tyr Lys Glu Gln Phe
            20                  25                  30

Leu Arg Leu Arg Lys Ser Ser Met Cys Gln Asn Gly Arg Asp Tyr Val
        35                  40                  45

Val Thr Lys Gln Pro Ser Ile Cys Leu Cys Ser Leu Glu Asp Phe Leu
    50                  55                  60

Cys Asp Phe Gly Tyr Tyr Arg Pro Glu Asn Asp Ser Lys Cys Val Glu
65                  70                  75                  80

Gln Pro Glu Leu Lys Gly His Asp Leu Glu Phe Cys Leu Tyr Gly Arg
                85                  90                  95

Glu Glu His Leu Thr Thr Asn Gly Tyr Arg Lys Ile Pro Gly Asp Lys
            100                 105                 110

Cys Gln Gly Gly Val Asn Pro Val Arg Glu Val Lys Asp Leu Lys Lys
        115                 120                 125

Lys Cys Thr Ser Asn Phe Leu Ser Pro Glu Lys Gln
    130                 135                 140

<210>  8
<211>  685
<212>  PRT
<213>  Artificial Sequence

<220>
<223>  IST218 after furin site plus HIS tag- isoform 1

<400>  8

Ser Ala Pro Gly Glu Asp Glu Glu Cys Gly Arg Val Arg Asp Phe Val
1               5                   10                  15

Ala Lys Leu Ala Asn Asn Thr His Gln His Val Phe Asp Asp Leu Arg
            20                  25                  30

Gly Ser Val Ser Leu Ser Trp Val Gly Asp Ser Thr Gly Val Ile Leu
        35                  40                  45

Val Leu Thr Thr Phe His Val Pro Leu Val Ile Met Thr Phe Gly Gln
    50                  55                  60

Ser Lys Leu Tyr Arg Ser Glu Asp Tyr Gly Lys Asn Phe Lys Asp Ile
65                  70                  75                  80

Thr Asp Leu Ile Asn Asn Thr Phe Ile Arg Thr Glu Phe Gly Met Ala
                85                  90                  95

Ile Gly Pro Glu Asn Ser Gly Lys Val Val Leu Thr Ala Glu Val Ser
            100                 105                 110

Gly Gly Ser Arg Gly Gly Arg Ile Phe Arg Ser Ser Asp Phe Ala Lys
        115                 120                 125

Asn Phe Val Gln Thr Asp Leu Pro Phe His Pro Leu Thr Gln Met Met
    130                 135                 140

Tyr Ser Pro Gln Asn Ser Asp Tyr Leu Leu Ala Leu Ser Thr Glu Asn
145                 150                 155                 160

Gly Leu Trp Val Ser Lys Asn Phe Gly Gly Lys Trp Glu Glu Ile His
                165                 170                 175

Lys Ala Val Cys Leu Ala Lys Trp Gly Ser Asp Asn Thr Ile Phe Phe
            180                 185                 190

Thr Thr Tyr Ala Asn Gly Ser Cys Lys Ala Asp Leu Gly Ala Leu Glu
        195                 200                 205

Leu Trp Arg Thr Ser Asp Leu Gly Lys Ser Phe Lys Thr Ile Gly Val
    210                 215                 220

Lys Ile Tyr Ser Phe Gly Leu Gly Gly Arg Phe Leu Phe Ala Ser Val
225                 230                 235                 240

Met Ala Asp Lys Asp Thr Thr Arg Arg Ile His Val Ser Thr Asp Gln
                245                 250                 255

Gly Asp Thr Trp Ser Met Ala Gln Leu Pro Ser Val Gly Gln Glu Gln
            260                 265                 270

Phe Tyr Ser Ile Leu Ala Ala Asn Asp Asp Met Val Phe Met His Val
        275                 280                 285

Asp Glu Pro Gly Asp Thr Gly Phe Gly Thr Ile Phe Thr Ser Asp Asp
    290                 295                 300

Arg Gly Ile Val Tyr Ser Lys Ser Leu Asp Arg His Leu Tyr Thr Thr
305                 310                 315                 320

Thr Gly Gly Glu Thr Asp Phe Thr Asn Val Thr Ser Leu Arg Gly Val
                325                 330                 335

Tyr Ile Thr Ser Val Leu Ser Glu Asp Asn Ser Ile Gln Thr Met Ile
            340                 345                 350

Thr Phe Asp Gln Gly Gly Arg Trp Thr His Leu Arg Lys Pro Glu Asn
        355                 360                 365

Ser Glu Cys Asp Ala Thr Ala Lys Asn Lys Asn Glu Cys Ser Leu His
    370                 375                 380

Ile His Ala Ser Tyr Ser Ile Ser Gln Lys Leu Asn Val Pro Met Ala
385                 390                 395                 400

Pro Leu Ser Glu Pro Asn Ala Val Gly Ile Val Ile Ala His Gly Ser
                405                 410                 415

Val Gly Asp Ala Ile Ser Val Met Val Pro Asp Val Tyr Ile Ser Asp
            420                 425                 430

Asp Gly Gly Tyr Ser Trp Thr Lys Met Leu Glu Gly Pro His Tyr Tyr
        435                 440                 445

Thr Ile Leu Asp Ser Gly Gly Ile Ile Val Ala Ile Glu His Ser Ser
    450                 455                 460

Arg Pro Ile Asn Val Ile Lys Phe Ser Thr Asp Glu Gly Gln Cys Trp
465                 470                 475                 480

Gln Thr Tyr Thr Phe Thr Arg Asp Pro Ile Tyr Phe Thr Gly Leu Ala
                485                 490                 495

Ser Glu Pro Gly Ala Arg Ser Met Asn Ile Ser Ile Trp Gly Phe Thr
            500                 505                 510

Glu Ser Phe Leu Thr Ser Gln Trp Val Ser Tyr Thr Ile Asp Phe Lys
        515                 520                 525

Asp Ile Leu Glu Arg Asn Cys Glu Glu Lys Asp Tyr Thr Ile Trp Leu
    530                 535                 540

Ala His Ser Thr Asp Pro Glu Asp Tyr Glu Asp Gly Cys Ile Leu Gly
545                 550                 555                 560

Tyr Lys Glu Gln Phe Leu Arg Leu Arg Lys Ser Ser Met Cys Gln Asn
                565                 570                 575

Gly Arg Asp Tyr Val Val Thr Lys Gln Pro Ser Ile Cys Leu Cys Ser
            580                 585                 590

Leu Glu Asp Phe Leu Cys Asp Phe Gly Tyr Tyr Arg Pro Glu Asn Asp
        595                 600                 605

Ser Lys Cys Val Glu Gln Pro Glu Leu Lys Gly His Asp Leu Glu Phe
    610                 615                 620

Cys Leu Tyr Gly Arg Glu Glu His Leu Thr Thr Asn Gly Tyr Arg Lys
625                 630                 635                 640

Ile Pro Gly Asp Lys Cys Gln Gly Gly Val Asn Pro Val Arg Glu Val

645                 650                 655

Lys Asp Leu Lys Lys Lys Cys Thr Ser Asn Phe Leu Ser Pro Glu Lys
            660                 665                 670

Gln Asn Ser Lys Ser Asn Ser His His His His His His
        675                 680                 685

<210>  9
<211>  679
<212>  PRT
<213>  Artificial Sequence

<220>
<223>  IST218 after furin site minus HIS tag- isoform 1

<400>  9

Ser Ala Pro Gly Glu Asp Glu Glu Cys Gly Arg Val Arg Asp Phe Val
1               5                   10                  15

Ala Lys Leu Ala Asn Asn Thr His Gln His Val Phe Asp Asp Leu Arg
            20                  25                  30

Gly Ser Val Ser Leu Ser Trp Val Gly Asp Ser Thr Gly Val Ile Leu
        35                  40                  45

Val Leu Thr Thr Phe His Val Pro Leu Val Ile Met Thr Phe Gly Gln
    50                  55                  60

Ser Lys Leu Tyr Arg Ser Glu Asp Tyr Gly Lys Asn Phe Lys Asp Ile
65                  70                  75                  80

Thr Asp Leu Ile Asn Asn Thr Phe Ile Arg Thr Glu Phe Gly Met Ala
                85                  90                  95

Ile Gly Pro Glu Asn Ser Gly Lys Val Val Leu Thr Ala Glu Val Ser
            100                 105                 110

Gly Gly Ser Arg Gly Gly Arg Ile Phe Arg Ser Ser Asp Phe Ala Lys
        115                 120                 125

Asn Phe Val Gln Thr Asp Leu Pro Phe His Pro Leu Thr Gln Met Met
    130                 135                 140

Tyr Ser Pro Gln Asn Ser Asp Tyr Leu Leu Ala Leu Ser Thr Glu Asn
145                 150                 155                 160

Gly Leu Trp Val Ser Lys Asn Phe Gly Gly Lys Trp Glu Glu Ile His
                165                 170                 175

Lys Ala Val Cys Leu Ala Lys Trp Gly Ser Asp Asn Thr Ile Phe Phe
        180                 185                 190

Thr Thr Tyr Ala Asn Gly Ser Cys Lys Ala Asp Leu Gly Ala Leu Glu
    195                 200                 205

Leu Trp Arg Thr Ser Asp Leu Gly Lys Ser Phe Lys Thr Ile Gly Val
    210                 215                 220

Lys Ile Tyr Ser Phe Gly Leu Gly Gly Arg Phe Leu Phe Ala Ser Val
225                 230                 235                 240

Met Ala Asp Lys Asp Thr Thr Arg Arg Ile His Val Ser Thr Asp Gln
                245                 250                 255

Gly Asp Thr Trp Ser Met Ala Gln Leu Pro Ser Val Gly Gln Glu Gln
            260                 265                 270

Phe Tyr Ser Ile Leu Ala Ala Asn Asp Asp Met Val Phe Met His Val
        275                 280                 285

Asp Glu Pro Gly Asp Thr Gly Phe Gly Thr Ile Phe Thr Ser Asp Asp
    290                 295                 300

Arg Gly Ile Val Tyr Ser Lys Ser Leu Asp Arg His Leu Tyr Thr Thr
305                 310                 315                 320

Thr Gly Gly Glu Thr Asp Phe Thr Asn Val Thr Ser Leu Arg Gly Val
                325                 330                 335

Tyr Ile Thr Ser Val Leu Ser Glu Asp Asn Ser Ile Gln Thr Met Ile
            340                 345                 350

Thr Phe Asp Gln Gly Gly Arg Trp Thr His Leu Arg Lys Pro Glu Asn
        355                 360                 365

Ser Glu Cys Asp Ala Thr Ala Lys Asn Lys Asn Glu Cys Ser Leu His
    370                 375                 380

Ile His Ala Ser Tyr Ser Ile Ser Gln Lys Leu Asn Val Pro Met Ala
385                 390                 395                 400

Pro Leu Ser Glu Pro Asn Ala Val Gly Ile Val Ile Ala His Gly Ser
                405                 410                 415

Val Gly Asp Ala Ile Ser Val Met Val Pro Asp Val Tyr Ile Ser Asp

            420                 425                 430

Asp Gly Gly Tyr Ser Trp Thr Lys Met Leu Glu Gly Pro His Tyr Tyr
        435                 440                 445

Thr Ile Leu Asp Ser Gly Gly Ile Ile Val Ala Ile Glu His Ser Ser
    450                 455                 460

Arg Pro Ile Asn Val Ile Lys Phe Ser Thr Asp Glu Gly Gln Cys Trp
465                 470                 475                 480

Gln Thr Tyr Thr Phe Thr Arg Asp Pro Ile Tyr Phe Thr Gly Leu Ala
                485                 490                 495

Ser Glu Pro Gly Ala Arg Ser Met Asn Ile Ser Ile Trp Gly Phe Thr
            500                 505                 510

Glu Ser Phe Leu Thr Ser Gln Trp Val Ser Tyr Thr Ile Asp Phe Lys
        515                 520                 525

Asp Ile Leu Glu Arg Asn Cys Glu Glu Lys Asp Tyr Thr Ile Trp Leu
    530                 535                 540

Ala His Ser Thr Asp Pro Glu Asp Tyr Glu Asp Gly Cys Ile Leu Gly
545                 550                 555                 560

Tyr Lys Glu Gln Phe Leu Arg Leu Arg Lys Ser Ser Met Cys Gln Asn
                565                 570                 575

Gly Arg Asp Tyr Val Val Thr Lys Gln Pro Ser Ile Cys Leu Cys Ser
            580                 585                 590

Leu Glu Asp Phe Leu Cys Asp Phe Gly Tyr Tyr Arg Pro Glu Asn Asp
        595                 600                 605

Ser Lys Cys Val Glu Gln Pro Glu Leu Lys Gly His Asp Leu Glu Phe
    610                 615                 620

Cys Leu Tyr Gly Arg Glu Glu His Leu Thr Thr Asn Gly Tyr Arg Lys
625                 630                 635                 640

Ile Pro Gly Asp Lys Cys Gln Gly Gly Val Asn Pro Val Arg Glu Val
                645                 650                 655

Lys Asp Leu Lys Lys Lys Cys Thr Ser Asn Phe Leu Ser Pro Glu Lys
            660                 665                 670

Gln Asn Ser Lys Ser Asn Ser
        675

<210>  10
<211>  680
<212>  PRT
<213>  Artificial Sequence

<220>
<223>  SKT1 after furin site plus HIS tag + TEV site-isoform 1

<400>  10

Ser Ala Pro Gly His His His His His His His Glu Asn Leu Tyr Phe
1               5                   10                  15

Gln Ser Glu Asp Glu Glu Cys Gly Arg Val Arg Asp Phe Val Ala Lys
            20                  25                  30

Leu Ala Asn Asn Thr His Gln His Val Phe Asp Asp Leu Arg Gly Ser
        35                  40                  45

Val Ser Leu Ser Trp Val Gly Asp Ser Thr Gly Val Ile Leu Val Leu
    50                  55                  60

Thr Thr Phe His Val Pro Leu Val Ile Met Thr Phe Gly Gln Ser Lys
65                  70                  75                  80

Leu Tyr Arg Ser Glu Asp Tyr Gly Lys Asn Phe Lys Asp Ile Thr Asp
                85                  90                  95

Leu Ile Asn Asn Thr Phe Ile Arg Thr Glu Phe Gly Met Ala Ile Gly
            100                 105                 110

Pro Glu Asn Ser Gly Lys Val Val Leu Thr Ala Glu Val Ser Gly Gly
        115                 120                 125

Ser Arg Gly Gly Arg Ile Phe Arg Ser Ser Asp Phe Ala Lys Asn Phe
    130                 135                 140

Val Gln Thr Asp Leu Pro Phe His Pro Leu Thr Gln Met Met Tyr Ser
145                 150                 155                 160

Pro Gln Asn Ser Asp Tyr Leu Leu Ala Leu Ser Thr Glu Asn Gly Leu
                165                 170                 175

Trp Val Ser Lys Asn Phe Gly Gly Lys Trp Glu Glu Ile His Lys Ala
            180                 185                 190

Val Cys Leu Ala Lys Trp Gly Ser Asp Asn Thr Ile Phe Phe Thr Thr

195                 200                 205

Tyr Ala Asn Gly Ser Cys Lys Ala Asp Leu Gly Ala Leu Glu Leu Trp
    210                 215                 220

Arg Thr Ser Asp Leu Gly Lys Ser Phe Lys Thr Ile Gly Val Lys Ile
225                 230                 235                 240

Tyr Ser Phe Gly Leu Gly Gly Arg Phe Leu Phe Ala Ser Val Met Ala
                245                 250                 255

Asp Lys Asp Thr Thr Arg Arg Ile His Val Ser Thr Asp Gln Gly Asp
            260                 265                 270

Thr Trp Ser Met Ala Gln Leu Pro Ser Val Gly Gln Glu Gln Phe Tyr
        275                 280                 285

Ser Ile Leu Ala Ala Asn Asp Asp Met Val Phe Met His Val Asp Glu
    290                 295                 300

Pro Gly Asp Thr Gly Phe Gly Thr Ile Phe Thr Ser Asp Asp Arg Gly
305                 310                 315                 320

Ile Val Tyr Ser Lys Ser Leu Asp Arg His Leu Tyr Thr Thr Thr Gly
                325                 330                 335

Gly Glu Thr Asp Phe Thr Asn Val Thr Ser Leu Arg Gly Val Tyr Ile
            340                 345                 350

Thr Ser Val Leu Ser Glu Asp Asn Ser Ile Gln Thr Met Ile Thr Phe
        355                 360                 365

Asp Gln Gly Gly Arg Trp Thr His Leu Arg Lys Pro Glu Asn Ser Glu
    370                 375                 380

Cys Asp Ala Thr Ala Lys Asn Lys Asn Glu Cys Ser Leu His Ile His
385                 390                 395                 400

Ala Ser Tyr Ser Ile Ser Gln Lys Leu Asn Val Pro Met Ala Pro Leu
                405                 410                 415

Ser Glu Pro Asn Ala Val Gly Ile Val Ile Ala His Gly Ser Val Gly
            420                 425                 430

Asp Ala Ile Ser Val Met Val Pro Asp Val Tyr Ile Ser Asp Asp Gly
        435                 440                 445

Gly Tyr Ser Trp Thr Lys Met Leu Glu Gly Pro His Tyr Tyr Thr Ile
    450                 455                 460

Leu Asp Ser Gly Gly Ile Ile Val Ala Ile Glu His Ser Ser Arg Pro
465                 470                 475                 480

Ile Asn Val Ile Lys Phe Ser Thr Asp Glu Gly Gln Cys Trp Gln Thr
                485                 490                 495

Tyr Thr Phe Thr Arg Asp Pro Ile Tyr Phe Thr Gly Leu Ala Ser Glu
            500                 505                 510

Pro Gly Ala Arg Ser Met Asn Ile Ser Ile Trp Gly Phe Thr Glu Ser
        515                 520                 525

Phe Leu Thr Ser Gln Trp Val Ser Tyr Thr Ile Asp Phe Lys Asp Ile
    530                 535                 540

Leu Glu Arg Asn Cys Glu Glu Lys Asp Tyr Thr Ile Trp Leu Ala His
545                 550                 555                 560

Ser Thr Asp Pro Glu Asp Tyr Glu Asp Gly Cys Ile Leu Gly Tyr Lys
                565                 570                 575

Glu Gln Phe Leu Arg Leu Arg Lys Ser Ser Met Cys Gln Asn Gly Arg
            580                 585                 590

Asp Tyr Val Val Thr Lys Gln Pro Ser Ile Cys Leu Cys Ser Leu Glu
        595                 600                 605

Asp Phe Leu Cys Asp Phe Gly Tyr Tyr Arg Pro Glu Asn Asp Ser Lys
    610                 615                 620

Cys Val Glu Gln Pro Glu Leu Lys Gly His Asp Leu Glu Phe Cys Leu
625                 630                 635                 640

Tyr Gly Arg Glu Glu His Leu Thr Thr Asn Gly Tyr Arg Lys Ile Pro
                645                 650                 655

Gly Asp Lys Cys Gln Gly Gly Val Asn Pro Val Arg Glu Val Lys Asp
            660                 665                 670

Leu Lys Lys Lys Cys Thr Ser Asn
        675                 680

<210>  11
<211>  662
<212>  PRT

<213>  Artificial Sequence

<220>
<223>  SKT1 after furin site minus HIS tag + TEV site- isoform 1

<400>  11

Glu Asp Glu Glu Cys Gly Arg Val Arg Asp Phe Val Ala Lys Leu Ala
1               5                   10                  15

Asn Asn Thr His Gln His Val Phe Asp Asp Leu Arg Gly Ser Val Ser
            20                  25                  30

Leu Ser Trp Val Gly Asp Ser Thr Gly Val Ile Leu Val Leu Thr Thr
        35                  40                  45

Phe His Val Pro Leu Val Ile Met Thr Phe Gly Gln Ser Lys Leu Tyr
    50                  55                  60

Arg Ser Glu Asp Tyr Gly Lys Asn Phe Lys Asp Ile Thr Asp Leu Ile
65                  70                  75                  80

Asn Asn Thr Phe Ile Arg Thr Glu Phe Gly Met Ala Ile Gly Pro Glu
                85                  90                  95

Asn Ser Gly Lys Val Val Leu Thr Ala Glu Val Ser Gly Gly Ser Arg
            100                 105                 110

Gly Gly Arg Ile Phe Arg Ser Ser Asp Phe Ala Lys Asn Phe Val Gln
        115                 120                 125

Thr Asp Leu Pro Phe His Pro Leu Thr Gln Met Met Tyr Ser Pro Gln
    130                 135                 140

Asn Ser Asp Tyr Leu Leu Ala Leu Ser Thr Glu Asn Gly Leu Trp Val
145                 150                 155                 160

Ser Lys Asn Phe Gly Gly Lys Trp Glu Glu Ile His Lys Ala Val Cys
                165                 170                 175

Leu Ala Lys Trp Gly Ser Asp Asn Thr Ile Phe Phe Thr Thr Tyr Ala
            180                 185                 190

Asn Gly Ser Cys Lys Ala Asp Leu Gly Ala Leu Glu Leu Trp Arg Thr
        195                 200                 205

Ser Asp Leu Gly Lys Ser Phe Lys Thr Ile Gly Val Lys Ile Tyr Ser
    210                 215                 220

Phe Gly Leu Gly Gly Arg Phe Leu Phe Ala Ser Val Met Ala Asp Lys
225                 230                 235                 240

Asp Thr Thr Arg Arg Ile His Val Ser Thr Asp Gln Gly Asp Thr Trp
                245                 250                 255

Ser Met Ala Gln Leu Pro Ser Val Gly Gln Glu Gln Phe Tyr Ser Ile
            260                 265                 270

Leu Ala Ala Asn Asp Asp Met Val Phe Met His Val Asp Glu Pro Gly
        275                 280                 285

Asp Thr Gly Phe Gly Thr Ile Phe Thr Ser Asp Asp Arg Gly Ile Val
    290                 295                 300

Tyr Ser Lys Ser Leu Asp Arg His Leu Tyr Thr Thr Thr Gly Gly Glu
305                 310                 315                 320

Thr Asp Phe Thr Asn Val Thr Ser Leu Arg Gly Val Tyr Ile Thr Ser
                325                 330                 335

Val Leu Ser Glu Asp Asn Ser Ile Gln Thr Met Ile Thr Phe Asp Gln
            340                 345                 350

Gly Gly Arg Trp Thr His Leu Arg Lys Pro Glu Asn Ser Glu Cys Asp
        355                 360                 365

Ala Thr Ala Lys Asn Lys Asn Glu Cys Ser Leu His Ile His Ala Ser
    370                 375                 380

Tyr Ser Ile Ser Gln Lys Leu Asn Val Pro Met Ala Pro Leu Ser Glu
385                 390                 395                 400

Pro Asn Ala Val Gly Ile Val Ile Ala His Gly Ser Val Gly Asp Ala
                405                 410                 415

Ile Ser Val Met Val Pro Asp Val Tyr Ile Ser Asp Asp Gly Gly Tyr
            420                 425                 430

Ser Trp Thr Lys Met Leu Glu Gly Pro His Tyr Tyr Thr Ile Leu Asp
        435                 440                 445

Ser Gly Gly Ile Ile Val Ala Ile Glu His Ser Ser Arg Pro Ile Asn
    450                 455                 460

Val Ile Lys Phe Ser Thr Asp Glu Gly Gln Cys Trp Gln Thr Tyr Thr
465                 470                 475                 480

Phe Thr Arg Asp Pro Ile Tyr Phe Thr Gly Leu Ala Ser Glu Pro Gly
                485                 490                 495

Ala Arg Ser Met Asn Ile Ser Ile Trp Gly Phe Thr Glu Ser Phe Leu
            500                 505                 510

Thr Ser Gln Trp Val Ser Tyr Thr Ile Asp Phe Lys Asp Ile Leu Glu
        515                 520                 525

Arg Asn Cys Glu Glu Lys Asp Tyr Thr Ile Trp Leu Ala His Ser Thr
    530                 535                 540

Asp Pro Glu Asp Tyr Glu Asp Gly Cys Ile Leu Gly Tyr Lys Glu Gln
545                 550                 555                 560

Phe Leu Arg Leu Arg Lys Ser Ser Met Cys Gln Asn Gly Arg Asp Tyr
                565                 570                 575

Val Val Thr Lys Gln Pro Ser Ile Cys Leu Cys Ser Leu Glu Asp Phe
            580                 585                 590

Leu Cys Asp Phe Gly Tyr Tyr Arg Pro Glu Asn Asp Ser Lys Cys Val
        595                 600                 605

Glu Gln Pro Glu Leu Lys Gly His Asp Leu Glu Phe Cys Leu Tyr Gly
    610                 615                 620

Arg Glu Glu His Leu Thr Thr Asn Gly Tyr Arg Lys Ile Pro Gly Asp
625                 630                 635                 640

Lys Cys Gln Gly Gly Val Asn Pro Val Arg Glu Val Lys Asp Leu Lys
                645                 650                 655

Lys Lys Cys Thr Ser Asn
            660

<210>  12
<211>  831
<212>  PRT
<213>  Homo sapiens

<400>  12

<210>  13
<211>  830
<212>  PRT
<213>  Homo sapiens

<400>  13
   Met Glu Arg Pro Trp Gly Ala Ala Asp Gly Leu Ser Arg Trp Pro His
   1               5                   10                  15
   Gly Leu Gly Leu Leu Leu Leu Leu Gln Leu Leu Pro Pro Ser Thr Leu
               20                  25                  30
   Ser Gln Asp Arg Leu Asp Ala Pro Pro Pro Pro Ala Ala Pro Leu Pro
           35                  40                  45
   Arg Trp Ser Gly Pro Ile Gly Val Ser Trp Gly Leu Arg Ala Ala Ala
       50                  55                  60
   Ala Gly Gly Ala Phe Pro Arg Gly Gly Arg Trp Arg Arg Ser Ala Pro
   65                  70                  75                  80
   Gly Glu Asp Glu Glu Cys Gly Arg Val Arg Asp Phe Val Ala Lys Leu
                   85                  90                  95
   Ala Asn Asn Thr His Gln His Val Phe Asp Asp Leu Arg Gly Ser Val
               100                 105                 110
   Ser Leu Ser Trp Val Gly Asp Ser Thr Gly Val Ile Leu Val Leu Thr
           115                 120                 125
   Thr Phe His Val Pro Leu Val Ile Met Thr Phe Gly Gln Ser Lys Leu
       130                 135                 140
   Tyr Arg Ser Glu Asp Tyr Gly Lys Asn Phe Lys Asp Ile Thr Asp Leu
   145                 150                 155                 160
   Ile Asn Asn Thr Phe Ile Arg Thr Glu Phe Gly Met Ala Ile Gly Pro
                   165                 170                 175
   Glu Asn Ser Gly Lys Val Val Leu Thr Ala Glu Val Ser Gly Gly Ser
               180                 185                 190
   Arg Gly Gly Arg Ile Phe Arg Ser Ser Asp Phe Ala Lys Asn Phe Val
           195                 200                 205
   Gln Thr Asp Leu Pro Phe His Pro Leu Thr Gln Met Met Tyr Ser Pro
       210                 215                 220
   Gln Asn Ser Asp Tyr Leu Leu Ala Leu Ser Thr Glu Asn Gly Leu Trp
   225                 230                 235                 240
   Val Ser Lys Asn Phe Gly Gly Lys Trp Glu Glu Ile His Lys Ala Val
                   245                 250                 255
   Cys Leu Ala Lys Trp Gly Ser Asp Asn Thr Ile Phe Phe Thr Thr Tyr
               260                 265                 270
   Ala Asn Gly Ser Cys Thr Asp Leu Gly Ala Leu Glu Leu Trp Arg Thr
           275                 280                 285
   Ser Asp Leu Gly Lys Ser Phe Lys Thr Ile Gly Val Lys Ile Tyr Ser
       290                 295                 300
   Phe Gly Leu Gly Gly Arg Phe Leu Phe Ala Ser Val Met Ala Asp Lys
   305                 310                 315                 320
   Asp Thr Thr Arg Arg Ile His Val Ser Thr Asp Gln Gly Asp Thr Trp
                   325                 330                 335
   Ser Met Ala Gln Leu Pro Ser Val Gly Gln Glu Gln Phe Tyr Ser Ile
               340                 345                 350
   Leu Ala Ala Asn Asp Asp Met Val Phe Met His Val Asp Glu Pro Gly
           355                 360                 365
   Asp Thr Gly Phe Gly Thr Ile Phe Thr Ser Asp Asp Arg Gly Ile Val
       370                 375                 380
   Tyr Ser Lys Ser Leu Asp Arg His Leu Tyr Thr Thr Thr Gly Gly Glu
   385                 390                 395                 400
   Thr Asp Phe Thr Asn Val Thr Ser Leu Arg Gly Val Tyr Ile Thr Ser
                   405                 410                 415
   Val Leu Ser Glu Asp Asn Ser Ile Gln Thr Met Ile Thr Phe Asp Gln
               420                 425                 430
   Gly Gly Arg Trp Thr His Leu Arg Lys Pro Glu Asn Ser Glu Cys Asp
           435                 440                 445
   Ala Thr Ala Lys Asn Lys Asn Glu Cys Ser Leu His Ile His Ala Ser
       450                 455                 460
   Tyr Ser Ile Ser Gln Lys Leu Asn Val Pro Met Ala Pro Leu Ser Glu
   465                 470                 475                 480
   Pro Asn Ala Val Gly Ile Val Ile Ala His Gly Ser Val Gly Asp Ala
                   485                 490                 495
   Ile Ser Val Met Val Pro Asp Val Tyr Ile Ser Asp Asp Gly Gly Tyr
               500                 505                 510
   Ser Trp Thr Lys Met Leu Glu Gly Pro His Tyr Tyr Thr Ile Leu Asp
           515                 520                 525
   Ser Gly Gly Ile Ile Val Ala Ile Glu His Ser Ser Arg Pro Ile Asn
       530                 535                 540
   Val Ile Lys Phe Ser Thr Asp Glu Gly Gln Cys Trp Gln Thr Tyr Thr
   545                 550                 555                 560
   Phe Thr Arg Asp Pro Ile Tyr Phe Thr Gly Leu Ala Ser Glu Pro Gly
                   565                 570                 575
   Ala Arg Ser Met Asn Ile Ser Ile Trp Gly Phe Thr Glu Ser Phe Leu
               580                 585                 590
   Thr Ser Gln Trp Val Ser Tyr Thr Ile Asp Phe Lys Asp Ile Leu Glu
           595                 600                 605
   Arg Asn Cys Glu Glu Lys Asp Tyr Thr Ile Trp Leu Ala His Ser Thr
       610                 615                 620
   Asp Pro Glu Asp Tyr Glu Asp Gly Cys Ile Leu Gly Tyr Lys Glu Gln
   625                 630                 635                 640
   Phe Leu Arg Leu Arg Lys Ser Ser Val Cys Gln Asn Gly Arg Asp Tyr
                   645                 650                 655
   Val Val Thr Lys Gln Pro Ser Ile Cys Leu Cys Ser Leu Glu Asp Phe
               660                 665                 670
   Leu Cys Asp Phe Gly Tyr Tyr Arg Pro Glu Asn Asp Ser Lys Cys Val
           675                 680                 685
   Glu Gln Pro Glu Leu Lys Gly His Asp Leu Glu Phe Cys Leu Tyr Gly
       690                 695                 700
   Arg Glu Glu His Leu Thr Thr Asn Gly Tyr Arg Lys Ile Pro Gly Asp
   705                 710                 715                 720
   Lys Cys Gln Gly Gly Val Asn Pro Val Arg Glu Val Lys Asp Leu Lys
                   725                 730                 735
   Lys Lys Cys Thr Ser Asn Phe Leu Ser Pro Glu Lys Gln Asn Ser Lys
               740                 745                 750
   Ser Asn Ser Val Pro Ile Ile Leu Ala Ile Val Gly Leu Met Leu Val
           755                 760                 765
   Thr Val Val Ala Gly Val Leu Ile Val Lys Lys Tyr Val Cys Gly Gly
       770                 775                 780
   Arg Phe Leu Val His Arg Tyr Ser Val Leu Gln Gln His Ala Glu Ala
   785                 790                 795                 800
   Asn Gly Val Asp Gly Val Asp Ala Leu Asp Thr Ala Ser His Thr Asn
                   805                 810                 815
   Lys Ser Gly Tyr His Asp Asp Ser Asp Glu Asp Leu Leu Glu
               820                 825                 830

<210>  14
<211>  539
<212>  PRT
<213>  Artificial Sequence

<220>
<223>  VPS10p Domain- isoform 2

<400>  14

Arg Gly Gly Arg Trp Arg Arg Ser Ala Pro Gly Glu Asp Glu Glu Cys
1               5                   10                  15

Gly Arg Val Arg Asp Phe Val Ala Lys Leu Ala Asn Asn Thr His Gln
            20                  25                  30

His Val Phe Asp Asp Leu Arg Gly Ser Val Ser Leu Ser Trp Val Gly
        35                  40                  45

Asp Ser Thr Gly Val Ile Leu Val Leu Thr Thr Phe His Val Pro Leu
    50                  55                  60

Val Ile Met Thr Phe Gly Gln Ser Lys Leu Tyr Arg Ser Glu Asp Tyr
65                  70                  75                  80

Gly Lys Asn Phe Lys Asp Ile Thr Asp Leu Ile Asn Asn Thr Phe Ile
                85                  90                  95

Arg Thr Glu Phe Gly Met Ala Ile Gly Pro Glu Asn Ser Gly Lys Val
            100                 105                 110

Val Leu Thr Ala Glu Val Ser Gly Gly Ser Arg Gly Gly Arg Ile Phe
        115                 120                 125

Arg Ser Ser Asp Phe Ala Lys Asn Phe Val Gln Thr Asp Leu Pro Phe
    130                 135                 140

His Pro Leu Thr Gln Met Met Tyr Ser Pro Gln Asn Ser Asp Tyr Leu
145                 150                 155                 160

Leu Ala Leu Ser Thr Glu Asn Gly Leu Trp Val Ser Lys Asn Phe Gly
                165                 170                 175

Gly Lys Trp Glu Glu Ile His Lys Ala Val Cys Leu Ala Lys Trp Gly
            180                 185                 190

Ser Asp Asn Thr Ile Phe Phe Thr Thr Tyr Ala Asn Gly Ser Cys Thr
        195                 200                 205

Asp Leu Gly Ala Leu Glu Leu Trp Arg Thr Ser Asp Leu Gly Lys Ser
    210                 215                 220

Phe Lys Thr Ile Gly Val Lys Ile Tyr Ser Phe Gly Leu Gly Gly Arg
225                 230                 235                 240

Phe Leu Phe Ala Ser Val Met Ala Asp Lys Asp Thr Thr Arg Arg Ile
                245                 250                 255

His Val Ser Thr Asp Gln Gly Asp Thr Trp Ser Met Ala Gln Leu Pro
            260                 265                 270

Ser Val Gly Gln Glu Gln Phe Tyr Ser Ile Leu Ala Ala Asn Asp Asp
        275                 280                 285

Met Val Phe Met His Val Asp Glu Pro Gly Asp Thr Gly Phe Gly Thr
    290                 295                 300

Ile Phe Thr Ser Asp Asp Arg Gly Ile Val Tyr Ser Lys Ser Leu Asp
305                 310                 315                 320

Arg His Leu Tyr Thr Thr Thr Gly Gly Glu Thr Asp Phe Thr Asn Val
                325                 330                 335

Thr Ser Leu Arg Gly Val Tyr Ile Thr Ser Val Leu Ser Glu Asp Asn
            340                 345                 350

Ser Ile Gln Thr Met Ile Thr Phe Asp Gln Gly Gly Arg Trp Thr His
        355                 360                 365

Leu Arg Lys Pro Glu Asn Ser Glu Cys Asp Ala Thr Ala Lys Asn Lys
    370                 375                 380

Asn Glu Cys Ser Leu His Ile His Ala Ser Tyr Ser Ile Ser Gln Lys
385                 390                 395                 400

Leu Asn Val Pro Met Ala Pro Leu Ser Glu Pro Asn Ala Val Gly Ile
                405                 410                 415

Val Ile Ala His Gly Ser Val Gly Asp Ala Ile Ser Val Met Val Pro
            420                 425                 430

Asp Val Tyr Ile Ser Asp Asp Gly Gly Tyr Ser Trp Thr Lys Met Leu
        435                 440                 445

Glu Gly Pro His Tyr Tyr Thr Ile Leu Asp Ser Gly Gly Ile Ile Val
    450                 455                 460

Ala Ile Glu His Ser Ser Arg Pro Ile Asn Val Ile Lys Phe Ser Thr
465                 470                 475                 480

Asp Glu Gly Gln Cys Trp Gln Thr Tyr Thr Phe Thr Arg Asp Pro Ile
                485                 490                 495

Tyr Phe Thr Gly Leu Ala Ser Glu Pro Gly Ala Arg Ser Met Asn Ile
            500                 505                 510

Ser Ile Trp Gly Phe Thr Glu Ser Phe Leu Thr Ser Gln Trp Val Ser
        515                 520                 525

Tyr Thr Ile Asp Phe Lys Asp Ile Leu Glu Arg
    530                 535

<210>  15
<211>  596
<212>  PRT
<213>  Artificial Sequence

<220>
<223>  VPS10p Domain + 10CCa Domain- isoform 2

<400>  15

Arg Gly Gly Arg Trp Arg Arg Ser Ala Pro Gly Glu Asp Glu Glu Cys
1               5                   10                  15

Gly Arg Val Arg Asp Phe Val Ala Lys Leu Ala Asn Asn Thr His Gln
            20                  25                  30

His Val Phe Asp Asp Leu Arg Gly Ser Val Ser Leu Ser Trp Val Gly
        35                  40                  45

Asp Ser Thr Gly Val Ile Leu Val Leu Thr Thr Phe His Val Pro Leu
    50                  55                  60

Val Ile Met Thr Phe Gly Gln Ser Lys Leu Tyr Arg Ser Glu Asp Tyr
65                  70                  75                  80

Gly Lys Asn Phe Lys Asp Ile Thr Asp Leu Ile Asn Asn Thr Phe Ile
                85                  90                  95

Arg Thr Glu Phe Gly Met Ala Ile Gly Pro Glu Asn Ser Gly Lys Val
            100                 105                 110

Val Leu Thr Ala Glu Val Ser Gly Gly Ser Arg Gly Gly Arg Ile Phe
        115                 120                 125

Arg Ser Ser Asp Phe Ala Lys Asn Phe Val Gln Thr Asp Leu Pro Phe
    130                 135                 140

His Pro Leu Thr Gln Met Met Tyr Ser Pro Gln Asn Ser Asp Tyr Leu
145                 150                 155                 160

Leu Ala Leu Ser Thr Glu Asn Gly Leu Trp Val Ser Lys Asn Phe Gly
                165                 170                 175

Gly Lys Trp Glu Glu Ile His Lys Ala Val Cys Leu Ala Lys Trp Gly
            180                 185                 190

Ser Asp Asn Thr Ile Phe Phe Thr Thr Tyr Ala Asn Gly Ser Cys Thr
        195                 200                 205

Asp Leu Gly Ala Leu Glu Leu Trp Arg Thr Ser Asp Leu Gly Lys Ser
    210                 215                 220

Phe Lys Thr Ile Gly Val Lys Ile Tyr Ser Phe Gly Leu Gly Gly Arg
225                 230                 235                 240

Phe Leu Phe Ala Ser Val Met Ala Asp Lys Asp Thr Thr Arg Arg Ile
                245                 250                 255

His Val Ser Thr Asp Gln Gly Asp Thr Trp Ser Met Ala Gln Leu Pro
            260                 265                 270

Ser Val Gly Gln Glu Gln Phe Tyr Ser Ile Leu Ala Ala Asn Asp Asp
        275                 280                 285

Met Val Phe Met His Val Asp Glu Pro Gly Asp Thr Gly Phe Gly Thr
    290                 295                 300

Ile Phe Thr Ser Asp Asp Arg Gly Ile Val Tyr Ser Lys Ser Leu Asp
305                 310                 315                 320

Arg His Leu Tyr Thr Thr Thr Gly Gly Glu Thr Asp Phe Thr Asn Val
                325                 330                 335

Thr Ser Leu Arg Gly Val Tyr Ile Thr Ser Val Leu Ser Glu Asp Asn
            340                 345                 350

Ser Ile Gln Thr Met Ile Thr Phe Asp Gln Gly Gly Arg Trp Thr His
        355                 360                 365

Leu Arg Lys Pro Glu Asn Ser Glu Cys Asp Ala Thr Ala Lys Asn Lys
    370                 375                 380

Asn Glu Cys Ser Leu His Ile His Ala Ser Tyr Ser Ile Ser Gln Lys
385                 390                 395                 400

Leu Asn Val Pro Met Ala Pro Leu Ser Glu Pro Asn Ala Val Gly Ile
                405                 410                 415

Val Ile Ala His Gly Ser Val Gly Asp Ala Ile Ser Val Met Val Pro
            420                 425                 430

Asp Val Tyr Ile Ser Asp Asp Gly Gly Tyr Ser Trp Thr Lys Met Leu
        435                 440                 445

Glu Gly Pro His Tyr Tyr Thr Ile Leu Asp Ser Gly Gly Ile Ile Val
    450                 455                 460

Ala Ile Glu His Ser Ser Arg Pro Ile Asn Val Ile Lys Phe Ser Thr
465                 470                 475                 480

Asp Glu Gly Gln Cys Trp Gln Thr Tyr Thr Phe Thr Arg Asp Pro Ile

485                 490                 495

Tyr Phe Thr Gly Leu Ala Ser Glu Pro Gly Ala Arg Ser Met Asn Ile
            500                 505                 510

Ser Ile Trp Gly Phe Thr Glu Ser Phe Leu Thr Ser Gln Trp Val Ser
        515                 520                 525

Tyr Thr Ile Asp Phe Lys Asp Ile Leu Glu Arg Asn Cys Glu Glu Lys
    530                 535                 540

Asp Tyr Thr Ile Trp Leu Ala His Ser Thr Asp Pro Glu Asp Tyr Glu
545                 550                 555                 560

Asp Gly Cys Ile Leu Gly Tyr Lys Glu Gln Phe Leu Arg Leu Arg Lys
                565                 570                 575

Ser Ser Val Cys Gln Asn Gly Arg Asp Tyr Val Val Thr Lys Gln Pro
            580                 585                 590

Ser Ile Cys Leu
        595

<210>  16
<211>  622
<212>  PRT
<213>  Artificial Sequence

<220>
<223>  VPS10p Domain + 10CCb Domain- isoform 2

<400>  16

Arg Gly Gly Arg Trp Arg Arg Ser Ala Pro Gly Glu Asp Glu Glu Cys
1               5                   10                  15

Gly Arg Val Arg Asp Phe Val Ala Lys Leu Ala Asn Asn Thr His Gln
            20                  25                  30

His Val Phe Asp Asp Leu Arg Gly Ser Val Ser Leu Ser Trp Val Gly
        35                  40                  45

Asp Ser Thr Gly Val Ile Leu Val Leu Thr Thr Phe His Val Pro Leu
    50                  55                  60

Val Ile Met Thr Phe Gly Gln Ser Lys Leu Tyr Arg Ser Glu Asp Tyr
65                  70                  75                  80

Gly Lys Asn Phe Lys Asp Ile Thr Asp Leu Ile Asn Asn Thr Phe Ile
                85                  90                  95

Arg Thr Glu Phe Gly Met Ala Ile Gly Pro Glu Asn Ser Gly Lys Val
            100                 105                 110

Val Leu Thr Ala Glu Val Ser Gly Gly Ser Arg Gly Gly Arg Ile Phe
        115                 120                 125

Arg Ser Ser Asp Phe Ala Lys Asn Phe Val Gln Thr Asp Leu Pro Phe
    130                 135                 140

His Pro Leu Thr Gln Met Met Tyr Ser Pro Gln Asn Ser Asp Tyr Leu
145                 150                 155                 160

Leu Ala Leu Ser Thr Glu Asn Gly Leu Trp Val Ser Lys Asn Phe Gly
                165                 170                 175

Gly Lys Trp Glu Glu Ile His Lys Ala Val Cys Leu Ala Lys Trp Gly
            180                 185                 190

Ser Asp Asn Thr Ile Phe Phe Thr Thr Tyr Ala Asn Gly Ser Cys Thr
        195                 200                 205

Asp Leu Gly Ala Leu Glu Leu Trp Arg Thr Ser Asp Leu Gly Lys Ser
    210                 215                 220

Phe Lys Thr Ile Gly Val Lys Ile Tyr Ser Phe Gly Leu Gly Gly Arg
225                 230                 235                 240

Phe Leu Phe Ala Ser Val Met Ala Asp Lys Asp Thr Thr Arg Arg Ile
                245                 250                 255

His Val Ser Thr Asp Gln Gly Asp Thr Trp Ser Met Ala Gln Leu Pro
            260                 265                 270

Ser Val Gly Gln Glu Gln Phe Tyr Ser Ile Leu Ala Ala Asn Asp Asp
        275                 280                 285

Met Val Phe Met His Val Asp Glu Pro Gly Asp Thr Gly Phe Gly Thr
    290                 295                 300

Ile Phe Thr Ser Asp Asp Arg Gly Ile Val Tyr Ser Lys Ser Leu Asp
305                 310                 315                 320

Arg His Leu Tyr Thr Thr Thr Gly Gly Glu Thr Asp Phe Thr Asn Val
                325                 330                 335

Thr Ser Leu Arg Gly Val Tyr Ile Thr Ser Val Leu Ser Glu Asp Asn

340                 345                 350

Ser Ile Gln Thr Met Ile Thr Phe Asp Gln Gly Gly Arg Trp Thr His
        355                 360                 365

Leu Arg Lys Pro Glu Asn Ser Glu Cys Asp Ala Thr Ala Lys Asn Lys
    370                 375                 380

Asn Glu Cys Ser Leu His Ile His Ala Ser Tyr Ser Ile Ser Gln Lys
385                 390                 395                 400

Leu Asn Val Pro Met Ala Pro Leu Ser Glu Pro Asn Ala Val Gly Ile
                405                 410                 415

Val Ile Ala His Gly Ser Val Gly Asp Ala Ile Ser Val Met Val Pro
            420                 425                 430

Asp Val Tyr Ile Ser Asp Asp Gly Gly Tyr Ser Trp Thr Lys Met Leu
        435                 440                 445

Glu Gly Pro His Tyr Tyr Thr Ile Leu Asp Ser Gly Gly Ile Ile Val
    450                 455                 460

Ala Ile Glu His Ser Ser Arg Pro Ile Asn Val Ile Lys Phe Ser Thr
465                 470                 475                 480

Asp Glu Gly Gln Cys Trp Gln Thr Tyr Thr Phe Thr Arg Asp Pro Ile
                485                 490                 495

Tyr Phe Thr Gly Leu Ala Ser Glu Pro Gly Ala Arg Ser Met Asn Ile
            500                 505                 510

Ser Ile Trp Gly Phe Thr Glu Ser Phe Leu Thr Ser Gln Trp Val Ser
        515                 520                 525

Tyr Thr Ile Asp Phe Lys Asp Ile Leu Glu Arg Cys Ser Leu Glu Asp
    530                 535                 540

Phe Leu Cys Asp Phe Gly Tyr Tyr Arg Pro Glu Asn Asp Ser Lys Cys
545                 550                 555                 560

Val Glu Gln Pro Glu Leu Lys Gly His Asp Leu Glu Phe Cys Leu Tyr
                565                 570                 575

Gly Arg Glu Glu His Leu Thr Thr Asn Gly Tyr Arg Lys Ile Pro Gly
            580                 585                 590

Asp Lys Cys Gln Gly Gly Val Asn Pro Val Arg Glu Val Lys Asp Leu
        595                 600                 605

Lys Lys Lys Cys Thr Ser Asn Phe Leu Ser Pro Glu Lys Gln
    610                 615                 620

<210>  17
<211>  679
<212>  PRT
<213>  Artificial Sequence

<220>
<223>  VPS10p Domain + 10CCa Domain + 10CCb Domain- isoform 2

<400>  17

Arg Gly Gly Arg Trp Arg Arg Ser Ala Pro Gly Glu Asp Glu Glu Cys
1               5                   10                  15

Gly Arg Val Arg Asp Phe Val Ala Lys Leu Ala Asn Asn Thr His Gln
            20                  25                  30

His Val Phe Asp Asp Leu Arg Gly Ser Val Ser Leu Ser Trp Val Gly
        35                  40                  45

Asp Ser Thr Gly Val Ile Leu Val Leu Thr Thr Phe His Val Pro Leu
    50                  55                  60

Val Ile Met Thr Phe Gly Gln Ser Lys Leu Tyr Arg Ser Glu Asp Tyr
65                  70                  75                  80

Gly Lys Asn Phe Lys Asp Ile Thr Asp Leu Ile Asn Asn Thr Phe Ile
                85                  90                  95

Arg Thr Glu Phe Gly Met Ala Ile Gly Pro Glu Asn Ser Gly Lys Val
            100                 105                 110

Val Leu Thr Ala Glu Val Ser Gly Gly Ser Arg Gly Gly Arg Ile Phe
        115                 120                 125

Arg Ser Ser Asp Phe Ala Lys Asn Phe Val Gln Thr Asp Leu Pro Phe
    130                 135                 140

His Pro Leu Thr Gln Met Met Tyr Ser Pro Gln Asn Ser Asp Tyr Leu
145                 150                 155                 160

Leu Ala Leu Ser Thr Glu Asn Gly Leu Trp Val Ser Lys Asn Phe Gly
                165                 170                 175

Gly Lys Trp Glu Glu Ile His Lys Ala Val Cys Leu Ala Lys Trp Gly

            180                 185                 190

Ser Asp Asn Thr Ile Phe Phe Thr Thr Tyr Ala Asn Gly Ser Cys Thr
        195                 200                 205

Asp Leu Gly Ala Leu Glu Leu Trp Arg Thr Ser Asp Leu Gly Lys Ser
    210                 215                 220

Phe Lys Thr Ile Gly Val Lys Ile Tyr Ser Phe Gly Leu Gly Gly Arg
225                 230                 235                 240

Phe Leu Phe Ala Ser Val Met Ala Asp Lys Asp Thr Thr Arg Arg Ile
                245                 250                 255

His Val Ser Thr Asp Gln Gly Asp Thr Trp Ser Met Ala Gln Leu Pro
            260                 265                 270

Ser Val Gly Gln Glu Gln Phe Tyr Ser Ile Leu Ala Ala Asn Asp Asp
        275                 280                 285

Met Val Phe Met His Val Asp Glu Pro Gly Asp Thr Gly Phe Gly Thr
    290                 295                 300

Ile Phe Thr Ser Asp Asp Arg Gly Ile Val Tyr Ser Lys Ser Leu Asp
305                 310                 315                 320

Arg His Leu Tyr Thr Thr Thr Gly Gly Glu Thr Asp Phe Thr Asn Val
                325                 330                 335

Thr Ser Leu Arg Gly Val Tyr Ile Thr Ser Val Leu Ser Glu Asp Asn
            340                 345                 350

Ser Ile Gln Thr Met Ile Thr Phe Asp Gln Gly Gly Arg Trp Thr His
        355                 360                 365

Leu Arg Lys Pro Glu Asn Ser Glu Cys Asp Ala Thr Ala Lys Asn Lys
    370                 375                 380

Asn Glu Cys Ser Leu His Ile His Ala Ser Tyr Ser Ile Ser Gln Lys
385                 390                 395                 400

Leu Asn Val Pro Met Ala Pro Leu Ser Glu Pro Asn Ala Val Gly Ile
                405                 410                 415

Val Ile Ala His Gly Ser Val Gly Asp Ala Ile Ser Val Met Val Pro
            420                 425                 430

Asp Val Tyr Ile Ser Asp Asp Gly Gly Tyr Ser Trp Thr Lys Met Leu
        435                 440                 445

Glu Gly Pro His Tyr Tyr Thr Ile Leu Asp Ser Gly Gly Ile Ile Val
    450                 455                 460

Ala Ile Glu His Ser Ser Arg Pro Ile Asn Val Ile Lys Phe Ser Thr
465                 470                 475                 480

Asp Glu Gly Gln Cys Trp Gln Thr Tyr Thr Phe Thr Arg Asp Pro Ile
                485                 490                 495

Tyr Phe Thr Gly Leu Ala Ser Glu Pro Gly Ala Arg Ser Met Asn Ile
            500                 505                 510

Ser Ile Trp Gly Phe Thr Glu Ser Phe Leu Thr Ser Gln Trp Val Ser
        515                 520                 525

Tyr Thr Ile Asp Phe Lys Asp Ile Leu Glu Arg Asn Cys Glu Glu Lys
    530                 535                 540

Asp Tyr Thr Ile Trp Leu Ala His Ser Thr Asp Pro Glu Asp Tyr Glu
545                 550                 555                 560

Asp Gly Cys Ile Leu Gly Tyr Lys Glu Gln Phe Leu Arg Leu Arg Lys
                565                 570                 575

Ser Ser Val Cys Gln Asn Gly Arg Asp Tyr Val Val Thr Lys Gln Pro
            580                 585                 590

Ser Ile Cys Leu Cys Ser Leu Glu Asp Phe Leu Cys Asp Phe Gly Tyr
        595                 600                 605

Tyr Arg Pro Glu Asn Asp Ser Lys Cys Val Glu Gln Pro Glu Leu Lys
    610                 615                 620

Gly His Asp Leu Glu Phe Cys Leu Tyr Gly Arg Glu Glu His Leu Thr
625                 630                 635                 640

Thr Asn Gly Tyr Arg Lys Ile Pro Gly Asp Lys Cys Gln Gly Gly Val
                645                 650                 655

Asn Pro Val Arg Glu Val Lys Asp Leu Lys Lys Lys Cys Thr Ser Asn
            660                 665                 670

Phe Leu Ser Pro Glu Lys Gln
        675

<210>  18
<211>  57
<212>  PRT
<213>  Artificial Sequence

<220>
<223>  10CCa Domain- isoform 2

<400>  18

Asn Cys Glu Glu Lys Asp Tyr Thr Ile Trp Leu Ala His Ser Thr Asp
1               5                   10                  15

Pro Glu Asp Tyr Glu Asp Gly Cys Ile Leu Gly Tyr Lys Glu Gln Phe
            20                  25                  30

Leu Arg Leu Arg Lys Ser Ser Val Cys Gln Asn Gly Arg Asp Tyr Val
        35                  40                  45

Val Thr Lys Gln Pro Ser Ile Cys Leu
    50                  55


<210>  19
<211>  140
<212>  PRT
<213>  Artificial Sequence

<220>
<223>  10CCa Domain + 10CCb Domain- isoform 2

<400>  19

Asn Cys Glu Glu Lys Asp Tyr Thr Ile Trp Leu Ala His Ser Thr Asp
1               5                   10                  15

Pro Glu Asp Tyr Glu Asp Gly Cys Ile Leu Gly Tyr Lys Glu Gln Phe
            20                  25                  30

Leu Arg Leu Arg Lys Ser Ser Val Cys Gln Asn Gly Arg Asp Tyr Val
        35                  40                  45

Val Thr Lys Gln Pro Ser Ile Cys Leu Cys Ser Leu Glu Asp Phe Leu
    50                  55                  60

Cys Asp Phe Gly Tyr Tyr Arg Pro Glu Asn Asp Ser Lys Cys Val Glu
65                  70                  75                  80

Gln Pro Glu Leu Lys Gly His Asp Leu Glu Phe Cys Leu Tyr Gly Arg
                85                  90                  95

Glu Glu His Leu Thr Thr Asn Gly Tyr Arg Lys Ile Pro Gly Asp Lys
            100                 105                 110

Cys Gln Gly Gly Val Asn Pro Val Arg Glu Val Lys Asp Leu Lys Lys
        115                 120                 125

Lys Cys Thr Ser Asn Phe Leu Ser Pro Glu Lys Gln
    130                 135                 140

<210>  20
<211>  684
<212>  PRT
<213>  Artificial Sequence

<220>
<223>  IST218 after furin site plus HIS tag- isoform 2

<400>  20

Ser Ala Pro Gly Glu Asp Glu Glu Cys Gly Arg Val Arg Asp Phe Val
1               5                   10                  15

Ala Lys Leu Ala Asn Asn Thr His Gln His Val Phe Asp Asp Leu Arg
            20                  25                  30

Gly Ser Val Ser Leu Ser Trp Val Gly Asp Ser Thr Gly Val Ile Leu
        35                  40                  45

Val Leu Thr Thr Phe His Val Pro Leu Val Ile Met Thr Phe Gly Gln
    50                  55                  60

Ser Lys Leu Tyr Arg Ser Glu Asp Tyr Gly Lys Asn Phe Lys Asp Ile
65                  70                  75                  80

Thr Asp Leu Ile Asn Asn Thr Phe Ile Arg Thr Glu Phe Gly Met Ala
                85                  90                  95

Ile Gly Pro Glu Asn Ser Gly Lys Val Val Leu Thr Ala Glu Val Ser
            100                 105                 110

Gly Gly Ser Arg Gly Gly Arg Ile Phe Arg Ser Ser Asp Phe Ala Lys
        115                 120                 125

Asn Phe Val Gln Thr Asp Leu Pro Phe His Pro Leu Thr Gln Met Met
    130                 135                 140

Tyr Ser Pro Gln Asn Ser Asp Tyr Leu Leu Ala Leu Ser Thr Glu Asn
145                 150                 155                 160

Gly Leu Trp Val Ser Lys Asn Phe Gly Gly Lys Trp Glu Glu Ile His
                165                 170                 175

Lys Ala Val Cys Leu Ala Lys Trp Gly Ser Asp Asn Thr Ile Phe Phe
            180                 185                 190

Thr Thr Tyr Ala Asn Gly Ser Cys Thr Asp Leu Gly Ala Leu Glu Leu
        195                 200                 205

Trp Arg Thr Ser Asp Leu Gly Lys Ser Phe Lys Thr Ile Gly Val Lys
    210                 215                 220

Ile Tyr Ser Phe Gly Leu Gly Gly Arg Phe Leu Phe Ala Ser Val Met
225                 230                 235                 240

Ala Asp Lys Asp Thr Thr Arg Arg Ile His Val Ser Thr Asp Gln Gly
                245                 250                 255

Asp Thr Trp Ser Met Ala Gln Leu Pro Ser Val Gly Gln Glu Gln Phe
            260                 265                 270

Tyr Ser Ile Leu Ala Ala Asn Asp Asp Met Val Phe Met His Val Asp
        275                 280                 285

Glu Pro Gly Asp Thr Gly Phe Gly Thr Ile Phe Thr Ser Asp Asp Arg
    290                 295                 300

Gly Ile Val Tyr Ser Lys Ser Leu Asp Arg His Leu Tyr Thr Thr Thr
305                 310                 315                 320

Gly Gly Glu Thr Asp Phe Thr Asn Val Thr Ser Leu Arg Gly Val Tyr
                325                 330                 335

Ile Thr Ser Val Leu Ser Glu Asp Asn Ser Ile Gln Thr Met Ile Thr
            340                 345                 350

Phe Asp Gln Gly Gly Arg Trp Thr His Leu Arg Lys Pro Glu Asn Ser
        355                 360                 365

Glu Cys Asp Ala Thr Ala Lys Asn Lys Asn Glu Cys Ser Leu His Ile
    370                 375                 380

His Ala Ser Tyr Ser Ile Ser Gln Lys Leu Asn Val Pro Met Ala Pro
385                 390                 395                 400

Leu Ser Glu Pro Asn Ala Val Gly Ile Val Ile Ala His Gly Ser Val
                405                 410                 415

Gly Asp Ala Ile Ser Val Met Val Pro Asp Val Tyr Ile Ser Asp Asp
            420                 425                 430

Gly Gly Tyr Ser Trp Thr Lys Met Leu Glu Gly Pro His Tyr Tyr Thr
        435                 440                 445

Ile Leu Asp Ser Gly Gly Ile Ile Val Ala Ile Glu His Ser Ser Arg
    450                 455                 460

Pro Ile Asn Val Ile Lys Phe Ser Thr Asp Glu Gly Gln Cys Trp Gln
465                 470                 475                 480

Thr Tyr Thr Phe Thr Arg Asp Pro Ile Tyr Phe Thr Gly Leu Ala Ser
                485                 490                 495

Glu Pro Gly Ala Arg Ser Met Asn Ile Ser Ile Trp Gly Phe Thr Glu
            500                 505                 510

Ser Phe Leu Thr Ser Gln Trp Val Ser Tyr Thr Ile Asp Phe Lys Asp
        515                 520                 525

Ile Leu Glu Arg Asn Cys Glu Glu Lys Asp Tyr Thr Ile Trp Leu Ala
    530                 535                 540

His Ser Thr Asp Pro Glu Asp Tyr Glu Asp Gly Cys Ile Leu Gly Tyr
545                 550                 555                 560

Lys Glu Gln Phe Leu Arg Leu Arg Lys Ser Ser Val Cys Gln Asn Gly
                565                 570                 575

Arg Asp Tyr Val Val Thr Lys Gln Pro Ser Ile Cys Leu Cys Ser Leu
            580                 585                 590

Glu Asp Phe Leu Cys Asp Phe Gly Tyr Tyr Arg Pro Glu Asn Asp Ser
        595                 600                 605

Lys Cys Val Glu Gln Pro Glu Leu Lys Gly His Asp Leu Glu Phe Cys
    610                 615                 620

Leu Tyr Gly Arg Glu Glu His Leu Thr Thr Asn Gly Tyr Arg Lys Ile
625                 630                 635                 640

Pro Gly Asp Lys Cys Gln Gly Gly Val Asn Pro Val Arg Glu Val Lys
                645                 650                 655

Asp Leu Lys Lys Lys Cys Thr Ser Asn Phe Leu Ser Pro Glu Lys Gln
            660                 665                 670

Asn Ser Lys Ser Asn Ser His His His His His His

675                 680

<210>  21
<211>  678
<212>  PRT
<213>  Artificial Sequence

<220>
<223>  IST218 after furin site minus HIS tag- isoform 2

<400>  21

Ser Ala Pro Gly Glu Asp Glu Glu Cys Gly Arg Val Arg Asp Phe Val
1               5                   10                  15

Ala Lys Leu Ala Asn Asn Thr His Gln His Val Phe Asp Asp Leu Arg
            20                  25                  30

Gly Ser Val Ser Leu Ser Trp Val Gly Asp Ser Thr Gly Val Ile Leu
        35                  40                  45

Val Leu Thr Thr Phe His Val Pro Leu Val Ile Met Thr Phe Gly Gln
    50                  55                  60

Ser Lys Leu Tyr Arg Ser Glu Asp Tyr Gly Lys Asn Phe Lys Asp Ile
65                  70                  75                  80

Thr Asp Leu Ile Asn Asn Thr Phe Ile Arg Thr Glu Phe Gly Met Ala
                85                  90                  95

Ile Gly Pro Glu Asn Ser Gly Lys Val Val Leu Thr Ala Glu Val Ser
            100                 105                 110

Gly Gly Ser Arg Gly Gly Arg Ile Phe Arg Ser Ser Asp Phe Ala Lys
        115                 120                 125

Asn Phe Val Gln Thr Asp Leu Pro Phe His Pro Leu Thr Gln Met Met
    130                 135                 140

Tyr Ser Pro Gln Asn Ser Asp Tyr Leu Leu Ala Leu Ser Thr Glu Asn
145                 150                 155                 160

Gly Leu Trp Val Ser Lys Asn Phe Gly Gly Lys Trp Glu Glu Ile His
                165                 170                 175

Lys Ala Val Cys Leu Ala Lys Trp Gly Ser Asp Asn Thr Ile Phe Phe
            180                 185                 190

Thr Thr Tyr Ala Asn Gly Ser Cys Thr Asp Leu Gly Ala Leu Glu Leu
        195                 200                 205

Trp Arg Thr Ser Asp Leu Gly Lys Ser Phe Lys Thr Ile Gly Val Lys
    210                 215                 220

Ile Tyr Ser Phe Gly Leu Gly Gly Arg Phe Leu Phe Ala Ser Val Met
225                 230                 235                 240

Ala Asp Lys Asp Thr Thr Arg Arg Ile His Val Ser Thr Asp Gln Gly
                245                 250                 255

Asp Thr Trp Ser Met Ala Gln Leu Pro Ser Val Gly Gln Glu Gln Phe
            260                 265                 270

Tyr Ser Ile Leu Ala Ala Asn Asp Asp Met Val Phe Met His Val Asp
        275                 280                 285

Glu Pro Gly Asp Thr Gly Phe Gly Thr Ile Phe Thr Ser Asp Asp Arg
    290                 295                 300

Gly Ile Val Tyr Ser Lys Ser Leu Asp Arg His Leu Tyr Thr Thr Thr
305                 310                 315                 320

Gly Gly Glu Thr Asp Phe Thr Asn Val Thr Ser Leu Arg Gly Val Tyr
                325                 330                 335

Ile Thr Ser Val Leu Ser Glu Asp Asn Ser Ile Gln Thr Met Ile Thr
            340                 345                 350

Phe Asp Gln Gly Gly Arg Trp Thr His Leu Arg Lys Pro Glu Asn Ser
        355                 360                 365

Glu Cys Asp Ala Thr Ala Lys Asn Lys Asn Glu Cys Ser Leu His Ile
    370                 375                 380

His Ala Ser Tyr Ser Ile Ser Gln Lys Leu Asn Val Pro Met Ala Pro
385                 390                 395                 400

Leu Ser Glu Pro Asn Ala Val Gly Ile Val Ile Ala His Gly Ser Val
                405                 410                 415

Gly Asp Ala Ile Ser Val Met Val Pro Asp Val Tyr Ile Ser Asp Asp
            420                 425                 430

Gly Gly Tyr Ser Trp Thr Lys Met Leu Glu Gly Pro His Tyr Tyr Thr
        435                 440                 445

Ile Leu Asp Ser Gly Gly Ile Ile Val Ala Ile Glu His Ser Ser Arg

450                 455                 460

Pro Ile Asn Val Ile Lys Phe Ser Thr Asp Glu Gly Gln Cys Trp Gln
465                 470                 475                 480

Thr Tyr Thr Phe Thr Arg Asp Pro Ile Tyr Phe Thr Gly Leu Ala Ser
                485                 490                 495

Glu Pro Gly Ala Arg Ser Met Asn Ile Ser Ile Trp Gly Phe Thr Glu
            500                 505                 510

Ser Phe Leu Thr Ser Gln Trp Val Ser Tyr Thr Ile Asp Phe Lys Asp
        515                 520                 525

Ile Leu Glu Arg Asn Cys Glu Glu Lys Asp Tyr Thr Ile Trp Leu Ala
    530                 535                 540

His Ser Thr Asp Pro Glu Asp Tyr Glu Asp Gly Cys Ile Leu Gly Tyr
545                 550                 555                 560

Lys Glu Gln Phe Leu Arg Leu Arg Lys Ser Ser Val Cys Gln Asn Gly
                565                 570                 575

Arg Asp Tyr Val Val Thr Lys Gln Pro Ser Ile Cys Leu Cys Ser Leu
            580                 585                 590

Glu Asp Phe Leu Cys Asp Phe Gly Tyr Tyr Arg Pro Glu Asn Asp Ser
        595                 600                 605

Lys Cys Val Glu Gln Pro Glu Leu Lys Gly His Asp Leu Glu Phe Cys
    610                 615                 620

Leu Tyr Gly Arg Glu Glu His Leu Thr Thr Asn Gly Tyr Arg Lys Ile
625                 630                 635                 640

Pro Gly Asp Lys Cys Gln Gly Gly Val Asn Pro Val Arg Glu Val Lys
                645                 650                 655

Asp Leu Lys Lys Lys Cys Thr Ser Asn Phe Leu Ser Pro Glu Lys Gln
            660                 665                 670

Asn Ser Lys Ser Asn Ser
        675

<210>  22
<211>  679
<212>  PRT
<213>  Artificial Sequence

<220>
<223>  SKT1 after furin site plus HIS tag + TEV site- isoform 2

<400>  22

Ser Ala Pro Gly His His His His His His His Glu Asn Leu Tyr Phe
1               5                   10                  15

Gln Ser Glu Asp Glu Glu Cys Gly Arg Val Arg Asp Phe Val Ala Lys
            20                  25                  30

Leu Ala Asn Asn Thr His Gln His Val Phe Asp Asp Leu Arg Gly Ser
        35                  40                  45

Val Ser Leu Ser Trp Val Gly Asp Ser Thr Gly Val Ile Leu Val Leu
    50                  55                  60

Thr Thr Phe His Val Pro Leu Val Ile Met Thr Phe Gly Gln Ser Lys
65                  70                  75                  80

Leu Tyr Arg Ser Glu Asp Tyr Gly Lys Asn Phe Lys Asp Ile Thr Asp
                85                  90                  95

Leu Ile Asn Asn Thr Phe Ile Arg Thr Glu Phe Gly Met Ala Ile Gly
            100                 105                 110

Pro Glu Asn Ser Gly Lys Val Val Leu Thr Ala Glu Val Ser Gly Gly
        115                 120                 125

Ser Arg Gly Gly Arg Ile Phe Arg Ser Ser Asp Phe Ala Lys Asn Phe
    130                 135                 140

Val Gln Thr Asp Leu Pro Phe His Pro Leu Thr Gln Met Met Tyr Ser
145                 150                 155                 160

Pro Gln Asn Ser Asp Tyr Leu Leu Ala Leu Ser Thr Glu Asn Gly Leu
                165                 170                 175

Trp Val Ser Lys Asn Phe Gly Gly Lys Trp Glu Glu Ile His Lys Ala
            180                 185                 190

Val Cys Leu Ala Lys Trp Gly Ser Asp Asn Thr Ile Phe Phe Thr Thr
        195                 200                 205

Tyr Ala Asn Gly Ser Cys Thr Asp Leu Gly Ala Leu Glu Leu Trp Arg
    210                 215                 220

Thr Ser Asp Leu Gly Lys Ser Phe Lys Thr Ile Gly Val Lys Ile Tyr

225                 230                 235                 240

Ser Phe Gly Leu Gly Gly Arg Phe Leu Phe Ala Ser Val Met Ala Asp
                245                 250                 255

Lys Asp Thr Thr Arg Arg Ile His Val Ser Thr Asp Gln Gly Asp Thr
            260                 265                 270

Trp Ser Met Ala Gln Leu Pro Ser Val Gly Gln Glu Gln Phe Tyr Ser
        275                 280                 285

Ile Leu Ala Ala Asn Asp Asp Met Val Phe Met His Val Asp Glu Pro
    290                 295                 300

Gly Asp Thr Gly Phe Gly Thr Ile Phe Thr Ser Asp Asp Arg Gly Ile
305                 310                 315                 320

Val Tyr Ser Lys Ser Leu Asp Arg His Leu Tyr Thr Thr Thr Gly Gly
                325                 330                 335

Glu Thr Asp Phe Thr Asn Val Thr Ser Leu Arg Gly Val Tyr Ile Thr
            340                 345                 350

Ser Val Leu Ser Glu Asp Asn Ser Ile Gln Thr Met Ile Thr Phe Asp
        355                 360                 365

Gln Gly Gly Arg Trp Thr His Leu Arg Lys Pro Glu Asn Ser Glu Cys
    370                 375                 380

Asp Ala Thr Ala Lys Asn Lys Asn Glu Cys Ser Leu His Ile His Ala
385                 390                 395                 400

Ser Tyr Ser Ile Ser Gln Lys Leu Asn Val Pro Met Ala Pro Leu Ser
                405                 410                 415

Glu Pro Asn Ala Val Gly Ile Val Ile Ala His Gly Ser Val Gly Asp
            420                 425                 430

Ala Ile Ser Val Met Val Pro Asp Val Tyr Ile Ser Asp Asp Gly Gly
        435                 440                 445

Tyr Ser Trp Thr Lys Met Leu Glu Gly Pro His Tyr Tyr Thr Ile Leu
    450                 455                 460

Asp Ser Gly Gly Ile Ile Val Ala Ile Glu His Ser Ser Arg Pro Ile
465                 470                 475                 480

Asn Val Ile Lys Phe Ser Thr Asp Glu Gly Gln Cys Trp Gln Thr Tyr
                485                 490                 495

Thr Phe Thr Arg Asp Pro Ile Tyr Phe Thr Gly Leu Ala Ser Glu Pro
            500                 505                 510

Gly Ala Arg Ser Met Asn Ile Ser Ile Trp Gly Phe Thr Glu Ser Phe
        515                 520                 525

Leu Thr Ser Gln Trp Val Ser Tyr Thr Ile Asp Phe Lys Asp Ile Leu
    530                 535                 540

Glu Arg Asn Cys Glu Glu Lys Asp Tyr Thr Ile Trp Leu Ala His Ser
545                 550                 555                 560

Thr Asp Pro Glu Asp Tyr Glu Asp Gly Cys Ile Leu Gly Tyr Lys Glu
                565                 570                 575

Gln Phe Leu Arg Leu Arg Lys Ser Ser Val Cys Gln Asn Gly Arg Asp
            580                 585                 590

Tyr Val Val Thr Lys Gln Pro Ser Ile Cys Leu Cys Ser Leu Glu Asp
        595                 600                 605

Phe Leu Cys Asp Phe Gly Tyr Tyr Arg Pro Glu Asn Asp Ser Lys Cys
    610                 615                 620

Val Glu Gln Pro Glu Leu Lys Gly His Asp Leu Glu Phe Cys Leu Tyr
625                 630                 635                 640

Gly Arg Glu Glu His Leu Thr Thr Asn Gly Tyr Arg Lys Ile Pro Gly
                645                 650                 655

Asp Lys Cys Gln Gly Gly Val Asn Pro Val Arg Glu Val Lys Asp Leu
            660                 665                 670

Lys Lys Lys Cys Thr Ser Asn
        675

<210>  23
<211>  661
<212>  PRT
<213>  Artificial Sequence

<220>
<223>  SKT1 after furin site minus HIS tag + TEV site- isoform 2

<400>  23

Glu Asp Glu Glu Cys Gly Arg Val Arg Asp Phe Val Ala Lys Leu Ala

1               5                   10                  15

Asn Asn Thr His Gln His Val Phe Asp Asp Leu Arg Gly Ser Val Ser
            20                  25                  30

Leu Ser Trp Val Gly Asp Ser Thr Gly Val Ile Leu Val Leu Thr Thr
        35                  40                  45

Phe His Val Pro Leu Val Ile Met Thr Phe Gly Gln Ser Lys Leu Tyr
    50                  55                  60

Arg Ser Glu Asp Tyr Gly Lys Asn Phe Lys Asp Ile Thr Asp Leu Ile
65                  70                  75                  80

Asn Asn Thr Phe Ile Arg Thr Glu Phe Gly Met Ala Ile Gly Pro Glu
                85                  90                  95

Asn Ser Gly Lys Val Val Leu Thr Ala Glu Val Ser Gly Gly Ser Arg
            100                 105                 110

Gly Gly Arg Ile Phe Arg Ser Ser Asp Phe Ala Lys Asn Phe Val Gln
        115                 120                 125

Thr Asp Leu Pro Phe His Pro Leu Thr Gln Met Met Tyr Ser Pro Gln
    130                 135                 140

Asn Ser Asp Tyr Leu Leu Ala Leu Ser Thr Glu Asn Gly Leu Trp Val
145                 150                 155                 160

Ser Lys Asn Phe Gly Gly Lys Trp Glu Glu Ile His Lys Ala Val Cys
                165                 170                 175

Leu Ala Lys Trp Gly Ser Asp Asn Thr Ile Phe Phe Thr Thr Tyr Ala
            180                 185                 190

Asn Gly Ser Cys Thr Asp Leu Gly Ala Leu Glu Leu Trp Arg Thr Ser
        195                 200                 205

Asp Leu Gly Lys Ser Phe Lys Thr Ile Gly Val Lys Ile Tyr Ser Phe
    210                 215                 220

Gly Leu Gly Gly Arg Phe Leu Phe Ala Ser Val Met Ala Asp Lys Asp
225                 230                 235                 240

Thr Thr Arg Arg Ile His Val Ser Thr Asp Gln Gly Asp Thr Trp Ser
                245                 250                 255

Met Ala Gln Leu Pro Ser Val Gly Gln Glu Gln Phe Tyr Ser Ile Leu
            260                 265                 270

Ala Ala Asn Asp Asp Met Val Phe Met His Val Asp Glu Pro Gly Asp
        275                 280                 285

Thr Gly Phe Gly Thr Ile Phe Thr Ser Asp Asp Arg Gly Ile Val Tyr
    290                 295                 300

Ser Lys Ser Leu Asp Arg His Leu Tyr Thr Thr Thr Gly Gly Glu Thr
305                 310                 315                 320

Asp Phe Thr Asn Val Thr Ser Leu Arg Gly Val Tyr Ile Thr Ser Val
                325                 330                 335

Leu Ser Glu Asp Asn Ser Ile Gln Thr Met Ile Thr Phe Asp Gln Gly
            340                 345                 350

Gly Arg Trp Thr His Leu Arg Lys Pro Glu Asn Ser Glu Cys Asp Ala
        355                 360                 365

Thr Ala Lys Asn Lys Asn Glu Cys Ser Leu His Ile His Ala Ser Tyr
    370                 375                 380

Ser Ile Ser Gln Lys Leu Asn Val Pro Met Ala Pro Leu Ser Glu Pro
385                 390                 395                 400

Asn Ala Val Gly Ile Val Ile Ala His Gly Ser Val Gly Asp Ala Ile
                405                 410                 415

Ser Val Met Val Pro Asp Val Tyr Ile Ser Asp Asp Gly Gly Tyr Ser
            420                 425                 430

Trp Thr Lys Met Leu Glu Gly Pro His Tyr Tyr Thr Ile Leu Asp Ser
        435                 440                 445

Gly Gly Ile Ile Val Ala Ile Glu His Ser Ser Arg Pro Ile Asn Val
    450                 455                 460

Ile Lys Phe Ser Thr Asp Glu Gly Gln Cys Trp Gln Thr Tyr Thr Phe
465                 470                 475                 480

Thr Arg Asp Pro Ile Tyr Phe Thr Gly Leu Ala Ser Glu Pro Gly Ala
                485                 490                 495

Arg Ser Met Asn Ile Ser Ile Trp Gly Phe Thr Glu Ser Phe Leu Thr
            500                 505                 510

Ser Gln Trp Val Ser Tyr Thr Ile Asp Phe Lys Asp Ile Leu Glu Arg
        515                 520                 525

Asn Cys Glu Glu Lys Asp Tyr Thr Ile Trp Leu Ala His Ser Thr Asp
    530                 535                 540

Pro Glu Asp Tyr Glu Asp Gly Cys Ile Leu Gly Tyr Lys Glu Gln Phe
545                 550                 555                 560

Leu Arg Leu Arg Lys Ser Ser Val Cys Gln Asn Gly Arg Asp Tyr Val
                565                 570                 575

Val Thr Lys Gln Pro Ser Ile Cys Leu Cys Ser Leu Glu Asp Phe Leu
            580                 585                 590

Cys Asp Phe Gly Tyr Tyr Arg Pro Glu Asn Asp Ser Lys Cys Val Glu
        595                 600                 605

Gln Pro Glu Leu Lys Gly His Asp Leu Glu Phe Cys Leu Tyr Gly Arg
    610                 615                 620

Glu Glu His Leu Thr Thr Asn Gly Tyr Arg Lys Ile Pro Gly Asp Lys
625                 630                 635                 640

Cys Gln Gly Gly Val Asn Pro Val Arg Glu Val Lys Asp Leu Lys Lys
                645                 650                 655

Lys Cys Thr Ser Asn
            660

<210>  24
<211>  7
<212>  PRT
<213>  Artificial Sequence

<220>
<223>  TEV cleavage site option 1

<400>  24

Glu Asn Leu Tyr Phe Gln Gly
1               5

<210>  25
<211>  7
<212>  PRT
<213>  Artificial Sequence

<220>
<223>  TEV cleavage site option 2

<400>  25

Glu Asn Leu Tyr Phe Gln Ser
1               5


<210>  26
<211>  6
<212>  PRT
<213>  Artificial Sequence

<220>
<223>  C terminal HIS tag

<400>  26

His His His His His His
1               5


<210>  27
<211>  7
<212>  PRT
<213>  Artificial Sequence

<220>
<223>  N terminal HIS tag

<400>  27

His His His His His His His
1               5

## Description

### Field of invention

The present invention relates to pharmaceutical compositions for use in the treatment of diseases including obesity, insulin resistance and diabetes mellitus.

### Background

Obesity is a medical condition in which excess body fat has accumulated to the extent that it may have a negative impact on health. With 61.4 % of adults in England, for example, being overweight or obese, obesity represents a major public health issue. Obesity is known to increase the risk of a range of health problems, including insulin resistance and the subsequent development of type 2 diabetes mellitus. Insulin resistance occurs when an individual's cells fail to respond normally to the hormone insulin. To overcome this, the pancreas (the organ responsible for producing insulin) compensates by producing more insulin to meet the metabolic demand. Ultimately, the levels of insulin produced are no longer adequate and therefore cellular glucose uptake is impaired, leading to hyperglycaemia and the likely development of diabetes mellitus.

The above diseases are not only an economic burden in their own right but can lead to multiple secondary disease states, further exacerbating the problem. Additionally, current pharmaceutical interventions fail to display adequate efficacy in a large proportion of patients, highlighting the need for novel treatment strategies.

The vacuolar protein sorting 10p domain (Vps10pD) receptor family is a group of five type I membrane homologs, known to include Sort1 (sortilin), SorL1, SorCS1, SorCS2 and SorCS3. This domain family was characterised following the identification of the yeast-sorting protein, Vps10p, which transports enzymes from the *trans*-Golgi network to the vacuole. These proteins are characterised by a domain within their N terminus that represents a site for ligand binding. Functions known to be attributed to this family of receptors include protein trafficking, lipid metabolism and both intracellular and intercellular signalling in a range of cell types.

In addition to the Vps10pD, SorCS1, SorCS2, SorCS3 all contain a leucine-rich segment between the Vps10pD and the transmembrane domain. SorLA is further characterised by the presence of fibronectin-type III repeats, low-density lipoprotein receptor class A and B repeats and an EGF precursor-type repeat. The peptide sequence homology between SorCS1, SorCS2, SorCS3 and SorLA is high, which is in contrast to a very low homology of these receptors with sortilin (for example the homology between human sortilin and human SorCS1 in the Vpsp10 domain is 25 %).

WO 2010/142296 claims a SorCS1-like agent for treating insulin resistance and/or a disease associated with insulin resistance in an individual, wherein said agent is capable of binding to and sensitising the insulin receptor at a SorCS1 binding site. Additionally, WO 2013/156031 claims the use of SorCS1 and SorCS1-like agents in a method of reducing appetite, suppressing hunger, increasing metabolism and treating obesity.

Different splice variants of SorCS1 display distinct patterns of tissue distribution and have been found to be present in tissues involved in glucose metabolism processes, such as adipose tissue, skeletal muscle and β-cells of the pancreas. SorCS1 knock-out mice show a reduced response to insulin when compared to wild-type control mice. Additionally, in a mouse model of diabetes mellitus, the over-expression of soluble SorCS1 reduced plasma levels of both glucose and insulin.

Sortilin is known to be enriched in the nervous system, where it can regulate neuronal development, functionality and integrity, via the binding of neurotrophic factors, for example. It is also known that it plays an important role in controlling metabolic activities. Examples of the latter include the regulation of cholesterol metabolism and regulation of the trafficking of GLUT-4 containing vesicles (Kjolby et al. Curr Atherosclerosis Reports 17(4):496, 2015).

Previous work has suggested a role for sortilin in the cellular signalling pathways associated with diseases such as diabetes and obesity. Sortilin levels have been shown to be modulated by the level of inflammation associated with these diseases. The pro-inflammatory cytokine, TNFα, reduces both mRNA levels and protein levels of sortilin in cultured mouse and human adipocytes, as well as *in vivo* when injected into mice (Kaddai et al. Diabetologia 52: 932-40, 2009). Sortilin can also influence cytokine secretion: targeting sortilin in immune cells has been proposed to attenuate inflammation (Mortenson et al. J Clin Invest 124(12):5317-22, 2014). Additionally, US 2016/0331746 describes various scaffolds of small molecules capable of binding to the active site of sortilin. Sortilin is involved in the regulation of glucose uptake (Shi & Kandror. Developmental Cell 9:99-108, 2005) and the development of lipid disorder diseases (Gao et al. DNA and Cell Biology 36(12):1050-61, 2017).

Further, plasma sortilin levels have been reported to be a potential biomarker for identifying patients with either coronary heart disease or diabetes mellitus (Oh et al. Cardiovascular Diabetology 16:92, 2017). Patients that showed increased sortilin levels within their plasma, and therefore identifiable as suffering from the above conditions, also displayed enhanced glucose levels suggesting sortilin as a therapeutic target for treating these conditions.

The listing or discussion of an apparently prior-published document in this specification should not necessarily be taken as an acknowledgment that the document is part of the state of the art or is common general knowledge.

### Summary of Invention

The present invention is based on the surprising discovery by the inventors that fragments of sortilin, when administered to diabetic mice, are able to significantly lower blood glucose levels by increasing insulin sensitivity following glucose challenge. This is particularly surprising given the very low homology of sortilin to SorCS1 and related proteins, and the suggestion in the art that downregulation of sortilin may be desirable for treating conditions relating to insulin resistance.

Accordingly, the present invention provides a composition comprising a fragment, derivative or variant of sortilin, or a sortilin mimetic, for use in medicine, wherein the sortilin mimetic is not SorCS1, SorCS2, SorCS3 or SorLA. In particular, the composition may be for use in treating or preventing diabetes mellitus, insulin resistance, obesity, metabolic disorders, polycystic ovary syndrome, non-alcoholic fatty liver disease, retinopathy, neuropathy, nephropathy, Alzheimer's disease and/or cardiovascular disease in a subject in need thereof.

The invention further provides an isolated polypeptide comprising the amino acid sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22 or SEQ ID NO: 23, or an amino acid sequence having greater than 70 % homology to said amino acid sequence, or a fragment thereof.

Further provided are nucleic acids that encode the polypeptides of the invention and pharmaceutical compositions comprising the isolated polypeptides or nucleic acids of the invention and medical uses thereof.

The present invention also provides a method of manufacturing polypeptides or fragments thereof according to the invention comprising the step of expressing the nucleic acid of the invention in a host cell or other suitable expression system as indicated herein.

The invention will now be described in more detail with reference to the following figures and examples.

### Figures

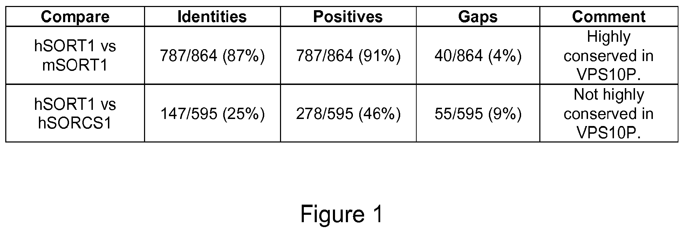

**Figure 1****: Alignment of hSORT1 and mSort1, hSORT1 and hSORCS1.**

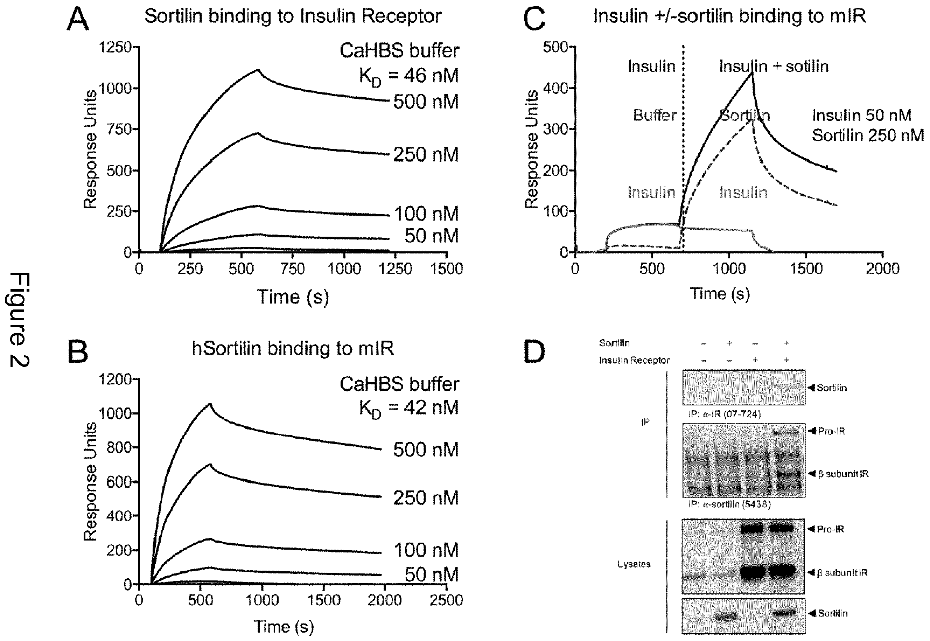

**Figure 2****: Sortilin and insulin receptor binding.** Surface plasmon resonance of sortilin (50-500 nM) binding to immobilized (A) human insulin receptor (0.0609 pmol/mm ²), and (B) murine insulin receptor (0.0607 pmol/mm²). Sortilin and insulin (C) is able to bind to the insulin receptor at the same time. (D) Co-immunoprecipitation with the insulin receptor and sortilin using DSP crosslinker. Immunoprecipitation of the insulin receptor resulted in co-precipitation of sortilin, and vice versa.

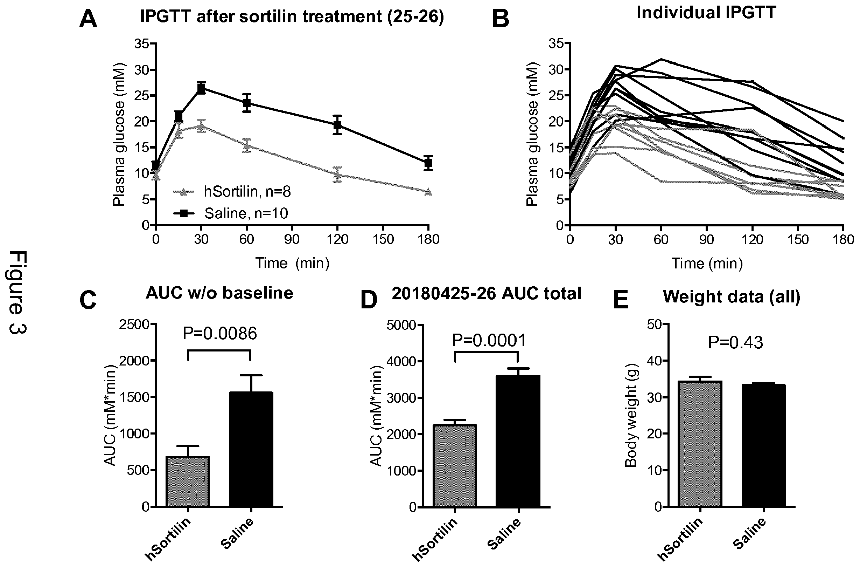

**Figure 3****. Effects of exogenous hSortilin given to *Lepr*^{*-*/*-*} (db/db) mice at t=-12 hrs and t=-1 hrs (100 µg/mouse x2).** Mice were challenged with an IP dose of glucose (1 mg/g BW) at t=0 min. (A) Plasma glucose levels in the intraperitoneal glucose tolerance test (IPGTT), (B) individual plasma glucose levels in IPGTT, (C) area under the curve (AUC) without baseline, (D) total AUC in IPGTT, and (E) body weight prior to conducting the experiment. Statistics: Student's t-test, and 2-way-ANOVA in panel A. Values are Mean ± SEM.

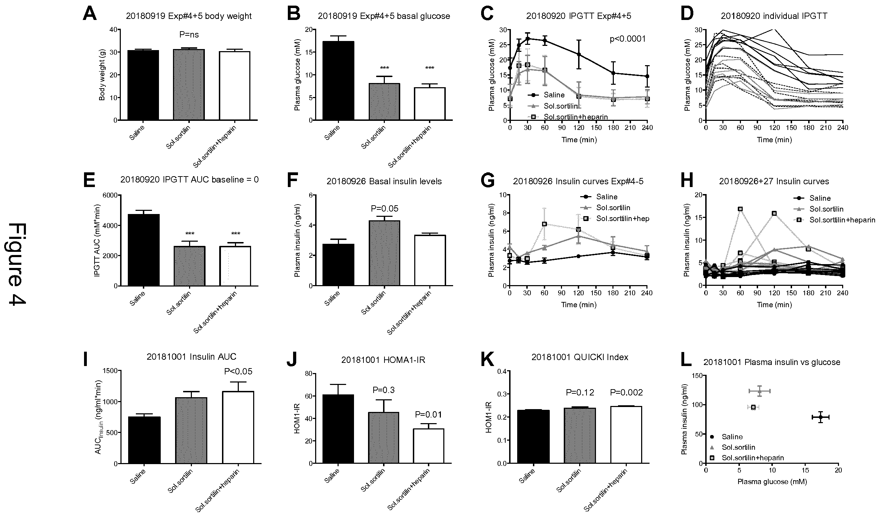

**Figure 4****. Effects of treatment with sortilin +/- heparin in diabetic db/db mice on IPGTT response.** (A) Initial bodyweight (g) (P=n.s), (B) basal glucose levels (mM) prior to IPGTT, (C) average IPGTT curves, and individual (D) curves. AUC (total) was lower in treated animals (E). Basal insulin levels (P=0.05 (soluble sortilin)) (F), and insulin IPGTT response curves (G-H), and insulin AUC (I). Insulin sensitivity index was calculated, HOMA1-IR (J), QUICKI Index (K), and plasma glucose vs insulin (L).

### Detailed Description

The following description is presented to enable any person skilled in the art to make and use the invention. Various modifications to the disclosed embodiments will be readily apparent to those skilled in the art.

In a first aspect the present invention provides a composition comprising a fragment, derivative or variant of sortilin, or a sortilin mimetic, for use in medicine, wherein the sortilin mimetic is not SorCS1, SorCS2, SorCS3 or SorLA.

Sortilin is a member of the Vps10p domain (Vps10pD) receptor family and has an amino acid sequence according to SEQ ID NO: 12 or SEQ ID NO: 13. Full length sortilin comprises a signal peptide, a propeptide, the Vps10p domain, a 10CC domain (10CCa + 10CCb), a transmembrane domain and a large cytoplasmic tail. The lumenal domain of sortilin has 6 potential *N*-linked glycosylation sites, whilst the cytoplasmic tail enables for the recruitment of various adapter proteins. Sortilin binds a vast number of ligands and membrane receptors and as a result engages in functions known to be important in cellular signalling and sorting.

The composition of the invention may include, in addition to the active pharmaceutical ingredient(s) (sortilin fragment, derivative, variant or mimetic), pharmaceutically acceptable carriers or excipients as would be understood by a person of skill in the art.

The present compositions may comprise sortilin fragments, derivatives or variants. By 'fragment' we intend any sequence of amino acids which partially constitute the full length amino acid sequence of sortilin. Therefore this includes any continuous length of amino acids with 100 % identity to a sortilin amino acid sequence. By 'derivative' we intend any sequence of natural or non-natural amino acids derived from reasonable modification of either the full length amino acid sequence of sortilin or the aforementioned sortilin fragments. Thus, the derivative may contain modified amino acids, D-amino acids, or amino acid mimics, which may be optimised for improved pharmacological properties, as would be understood by a person of skill in the art. By 'variant' we intend any form of sortilin or amino acid with high homology to sortilin and functional equivalent, which includes splice variants and homologues from organisms other than humans, for example, simian, murine or porcine homologues.

The present composition may comprise sortilin mimetics. The term 'sortilin mimetic' is intended to refer to any substance which exerts the same function as sortilin, sortilin fragments, derivatives or variants thereof. For example, such compounds may exhibit the same receptor binding capabilities as sortilin, even though they may not share structural homology to sortilin. However, "sortilin mimetic" does not include SorCS1, SorCS2, SorCS3 or SorLA. It is envisaged that these substances may include antibodies, nanobodies and aptamers which are able to interact with the sortilin binding epitope(s) within the insulin receptor. Methods for detecting such an interaction may involve techniques such as phage display, an *in vitro* screening technique designed for identifying ligands for proteins. The antibody used in the present invention may be, for example, a monoclonal antibody, a humanised antibody, a chimeric antibody, a recombinant antibody or a bispecifc antibody. By 'bispecific antibody' we intend an antibody that can simultaneously bind to two different antigens. The latter may be a suitable method to bridge the insulin receptor with endogenous sortilin. Fragments of said antibodies may also be used, specifically, antigen-binding fragments or single chain variable fragments. Preferred fragments will retain some or all the ability of an antibody to selectively bind to its target. By 'nanobodies' we intend the resulting protein based on single-domain antibody fragments that contain the unique structural and functional properties of naturally-occurring heavy chain only antibodies. By 'aptamer' we intend an oligonucleotide or peptide molecule capable of binding to a specific target molecule. Aptamers can bind to their target with high selectivity and specificity. Target recognition and binding may involve shape-dependent interactions as well as hydrophobic interactions, base-stacking and intercalation. Common targets for these molecules may include proteins, peptides, carbohydrates and small molecules. The methods by which the above antibodies, antibody fragments and aptamers are produced will be well known to the person skilled in the art.

It is envisaged that the present invention may useful for treating a range of diseases. The invention may be for use as a therapeutic and/or a prophylactic treatment. Specifically, the present invention is intended for use in diseases associated with insulin resistance. These diseases may be as a direct result of elevated blood glucose levels or as a secondary cause. Examples of such diseases include, but are not limited to, diabetes mellitus, obesity, metabolic disorders, polycystic ovary syndrome, non-alcoholic fatty liver disease, retinopathy, neuropathy, nephropathy, Alzheimer's disease, cardiovascular disease, stroke, coeliac disease, thyroid disease, mastopathy, muscular conditions, cirrhosis, hyperglycemia, hyperinsulinemia, arteriosclerosis, hypercholesterolemia, hypertriglyceridemia, dyslipidemia, hypercoagulability, hypertension, microalbuminura and any combinations thereof.

In one embodiment of the composition for use according to the invention, the sortilin fragment, derivative or variant comprises the VPS10p, 10CCa, or 10CCb domain. Also included are sortilin fragments, derivatives or variants that include any combination of the above domains, for example, VPS10p + 10CCa + 10CCb domain, VPS10p + 10CCa domain, VPS10p + 10CCb domain or 10CCa + 10CCb domain. The composition for use may comprise a dimer of any of the aforementioned polypeptides. By 'dimer' we intend a macromolecular complex formed by two single protein monomers, which may be expressed as a fusion protein.

The amino acid sequence of the Vps10p, 10CCa and 10CCb domain is provided herein as SEQ ID NO: 1, SEQ ID NO: 5, SEQ ID NO: 6, SEQ ID NO: 14 and SEQ ID NO: 18.

The amino acid sequence of the Vps10p + 10CCa + 10CCb domains, Vps10p + 10CCa domains, Vps10p + 10CCb domains and the 10CCa + 10CCb domains are provided herein as SEQ ID NO: 4, SEQ ID NO: 2, SEQ ID NO: 3, SEQ ID NO: 7, SEQ ID NO: 17, SEQ ID NO: 15, SEQ ID NO: 16 and SEQ ID NO: 19.

The above amino acid sequences may or may not include protein tags. The presence of said tags may result in superior expression and/or an easier purification process. One such example could be the use of a HIS-tag, located either at the C-terminal (SEQ ID NO: 26) or at the N-terminal (SEQ ID NO: 27) with an additional TEV cleavage site. C-terminal HIS tagged sortilin is designed to have the wild type N-terminal after furin cleavage (allowing for protein activation). N-terminal HIS tagged sortilin is designed to have an N-terminal HIS tag after furin-mediated cleavage of the propeptide in the Golgi. The HIS tag can be cleaved off using a TEV protease, the latter of which recognises a strict 7 amino acid sequence of ENLYFQ(G/S) (SEQ ID NOs: 24 and 25, respectively).

The amino acid sequence of the C-terminal HIS tagged sortilin is herein provided as SEQ ID NO: 8 and SEQ ID NO: 20, and the amino acid sequence of the N-terminal HIS tagged sortilin is herein provided as SEQ ID NO: 10 and SEQ ID NO: 22.

It is intended that the above amino acid sequences may comprise an additional moiety and/or undergo a modification process. The skilled person would recognise that these modifications may result in improvements across a number of parameters, for example, enhanced stability, enhanced efficacy, improved half-life and/or reduced toxicity levels. Suitable additional moieties may include linker molecules, such as glutamic acid. These linker molecules may be of varying lengths, for example 2-31 amino acids in length. Lipophilic groups may also be attached. Examples of such lipophilic groups include fatty acids, isoprenoids, sterols, phospholipids and glycosylphosphatidyl inositol anchors. Possible modifications may include PEGylation, glycosylation or acetylation. PEGylation may involve the covalent or non-covalent addition of single, or multiple, polyethylene glycol polymer chains to the amino acid sequence. Glycosylation may involve the addition of a carbohydrate which is covalently attached to the amino acid sequence and acetylation refers to the process of introducing an acetyl group into said sequence. Further modifications may include methods to reduce binding of said sequence to proteoglycans or sulphur containing polysaccharides and oligosaccharides. Such modifications may utilise sulfatases to result in desulphation of said molecules.

The present invention provides any sortilin fragment or polypeptide with at least 70 % homology to the aforementioned amino acid sequences. For example, the sortilin fragment or polypeptide of the invention may have at least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % homology to the isolated polypeptides of any of SEQ ID NOs: 1, 2, 3, 4, 5, 6, 7, 8, 9, 10, 11, 14, 15, 16, 17, 18, 19, 20, 21, 22 and/or 23. Further, also included are polypeptides with one or more conservative substitutions. By 'conservative substitution' we intend an amino acid replacement that changes one or more amino acids whilst resulting in a substance with similar biochemical properties. These biochemical properties may include charge, hydrophobicity and size, as would be understood by a person of skill in the art.

In an embodiment, the fragment, derivative or variant of sortilin, or sortilin mimetic may bind to an insulin receptor. It is preferred that the fragment, derivative or variant of sortilin, or sortilin mimetic binds to a human insulin receptor. Alternatively, or additionally, the fragment, derivative or variant of sortilin, or sortilin mimetic may bind to an insulin receptor from any mammal, for example, a mouse, guinea pig, rat, rabbit, dog, cat, horse, cow, sheep or pig insulin receptor.

It is intended that the sortilin fragments, derivatives, variants or sortilin mimetics of the present invention interact with the insulin receptor, resulting in improved blood glucose level control. The present inventors have demonstrated that sortilin binds to the human/mouse insulin receptor in a dose-dependent manner with high affinity, displaying a Kd value of approximately 40 nM. The latter allows for the desired therapeutic effect (a reduction in blood glucose levels) of the sortilin fragments, derivative, variants or sortilin mimetics to occur at low concentrations, thus minimising the chances of associated adverse effects. The inventors also show that the presence of insulin does not affect the binding between sortilin and the insulin receptor, instead forming a trimeric receptor complex, thus allowing for continued insulin-driven cell signalling. Consequently, the use of subcutaneously administered human soluble sortilin is shown to significantly improve glucose handling after a glucose challenge in a diabetic mouse strain. This effect can be observed within less than 12 hours of treatment, displaying the impressive fast-acting properties of the compositions and polypeptides of the present invention.

In a second aspect, the invention provides an isolated polypeptide comprising the amino acid sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22 or SEQ ID NO: 23, or an amino acid sequence having greater than 70 % homology to said amino acid sequence, or a fragment thereof.

In a further aspect, the invention provides a nucleic acid encoding any of the polypeptides as defined herein.

The invention also provides a nucleic acid that hybridises to a nucleic acid of the invention under low and/or high stringency conditions. Accordingly, the nucleic acid may be complementary to the nucleic acid encoding a polypeptide of the invention. By "high stringency conditions" we include high temperature and low salt content in hybridisation buffers. By "low stringency conditions" we include lower temperatures and high salt content in hybridisation buffers. The present invention may involve the use of siRNAs (commonly referred to as small interfering or silencing RNA) or Morpholino oligomers, directed against the nucleotide sequences or regulatory portions thereof *in vivo.* By 'siRNA' we intend a class of double-stranded RNA molecules, commonly 20-25 base pairs in length. siRNAs operate within the RNA interference pathway and therefore interfere with gene expression. Most commonly, siRNAs prevent translation for the degradation of mRNA. By 'Morpholino oligomers' we intend an oligomer molecule wherein the structure has DNA bases attached to a backbone of methylenemorpholine rings, linked through phosphorodiamidate groups. These molecules commonly act via blocking the access of other molecules to specific sequences of the RNA molecule. The use of these molecules would be particularly relevant in the instance where the translation of the protein was inhibited, thus triggering a feedback loop wherein the transcript levels were in fact enhanced, leading to increased protein levels of sortilin fragments, derivatives or variants. Alternatively, these genetic tools could be targeted to transcripts known to be associated with protein regulation, in particular degradation, thus leading to an upregulation of sortilin protein.

In a further aspect, the present invention provides an expression vector comprising a nucleic acid according to the invention. The expression vector may be used to manufacture the polypeptides of the invention *in vitro* (for subsequent formulation and delivery) or indeed to be delivered direct to the patient to be treated where the expression of the polypeptides of the invention will occur in the patient themselves. Thus, the expression vector may be used in gene therapy to treat a condition identified herein.

The vector may be any suitable vector for *ex vivo* or *in vivo* expression. For example, the vector may a vector suitable for use in human gene therapy. The vector may alternatively be any vector suitable for use for expression in bacteria, mammalian cell lines, plants, yeasts, protozoa or cell free expression systems. Suitable examples of vectors according to the invention are viruses or plasmids. Examples of appropriate viruses include, but are not limited to, retroviruses, adenoviruses, adeno-associated viruses and the herpes simplex virus. Non-viral vector systems may also be used, for example, lipid DNA complexes. The promoter used to achieve activation of said nucleotide sequence may be selected from the group comprising: lentivirus, adeno-associated virus (AAV), cytomegalovirus (CMV), Rous sarcoma virus (RSV), elongation factor 1 alpha (EF1α), Tet-regulatable promoter, platelet-derived growth factor beta (PDGFβ), murine leukemia virus long terminal repeat (Mo-MLV-L TR), phosphoglycerate kinase (PGK), Mx1 and calmodulin-dependent protein kinase II (CaMK II). Ideally, the promoter will be tissue-specific to restrict unwanted transgene expression and to facilitate persistent transgene expression, preferentially in liver, adipose tissue or muscle. Liver specific promoters may include albumin (Palb), α1-antitrypsin (Pa1AT), hemopexin (Phpx), apolipoprotein A2 (APOA2), serpin peptidase inhibitor (SERPINA1), cytochrome p450 (CYP3A4), microRNA 122 (MIR122). Adipose tissue specific promoters may include adiponectin (AdipoQ) and muscle specific promoters may include muscle creatine kinase (MCK), myosin heavy chain 6 (Myh6), myosin light chain 2 (MYL2) and (tropomyosin 3) TPM3.

In a further aspect, the present invention provides a host cell or cell free expression system comprising an expression vector according to the invention. This may be for purposes of producing said polypeptide or for the purpose of advanced cell therapy techniques. The latter may involve isolation and manipulation of a patient's own cells to produce a desired alteration, before re-delivery to the patient. The host cell may be mammalian, bacterial, yeast, plant or insect derived. 'Mammalian' is taken to include cells from human, feline, porcine, simian, canine, murine and rat origin and may include the use of muscle cells, hepatocytes, adipocytes and cells of the pancreas, such as α, β and δ cells. Conveniently Chinese hamster ovary (CHO) or Human embryonic kidney (HEK) cells may be utilised but any other suitable host cell may be used as would be understood by a person of skill in the art. Yeast systems which may be used include *Pichia pastoris, Kluyveromyces lactis* and *Saccharomyces cerevisiae.* Bacterial systems which would be appropriate for this purpose may include *Escherichia coli, Corynebacterium,* and *Pseudomonas fluorescens.* A cell free expression system may also be utilised wherein protein production is performed *in vitro,* using purified RNA polymerase, ribosomes, tRNA and ribonucleotides.

In a further aspect, the present invention provides a pharmaceutical composition comprising the isolated polypeptide or derivative or mimic, nucleic acid, expression vector or host cell of the preceding aspects and embodiments in combination with a pharmaceutically acceptable carrier or excipient. The excipients and carriers may enhance stability of the pharmaceutically active component, improve the biopharmaceutical profile and patient acceptability as well as simplify the manufacturing process, as would be understood by a person of skill in the art. Examples of suitable carriers or excipients include; sterile water, olive oil, ethyl oleate, glycols carbohydrates, especially monosaccharides such as fructose, glucose and galactose; non-reducing disaccharides such as sucrose, lactose and trehalose; non-reducing oligosaccharides such as raffinose and melezitose; non reducing starch derived polysaccharides products such as maltodextrins, dextrans and cyclodextrins; and non-reducing alditols such as mannitol and xylitol. Further suitable excipients include cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP). Mixtures of two or more of any of the above excipients are also envisaged. Specifically, it is envisaged that simple saline solutions may be used for this purpose. Formulations that the present invention may take include lyophilised formulations and depot formulations, whereby the depot injection is given subcutaneously, intradermal or intramuscularly. Specific encapsulation techniques may be used as a drug delivery device, for example, PLGA (polylactic-*co*-glycolic acid) microspheres.

The present invention also provides the pharmaceutical composition according to the preceding aspect for use in medicine. In particular, the pharmaceutical composition for use herein may be for use in treating, or in the prevention of, diabetes mellitus, insulin resistance, obesity, metabolic disorders, polycystic ovary syndrome, non-alcoholic fatty liver disease, retinopathy, neuropathy, nephropathy, Alzheimer's disease and/or cardiovascular disease in a subject in need thereof.

Pharmaceutical compositions may be in the form of liquids, solutions, suspensions, emulsions, elixirs, syrups, tablets, lozenges, granules, powders, capsules, cachets, pills, ampoules, suppositories, pessaries, ointments, gels, pastes, creams, sprays, mists, foams, lotions, pills, boluses, electuaries, or aerosols.

The pharmaceutical compositions may be prepared as suitable according to the intended route of administration. Possible routes of administration of said pharmaceutical composition include oral, sublingual, rectal, topical, parenteral and intravenous injection administration. Preferably, the chosen route of administration would be a subcutaneous injection, also known as SC, SQ, sub-cu, sub-Q or subcut. The site of injection may be the upper arm, the abdomen, the thigh, the upper back or the upper buttock.

The dose of the present invention is envisaged to be in the range of 0.01 to 1 mg/kg. The dose may be a daily dose of 0.05 mg/kg but ideally is administered once a week. The dosing regime may involve a single dose or multiple doses. It is understood that the exact dose and dosing regime will be dependent on the disease in question.

The present invention also provides for the use of a composition comprising a fragment, derivative or variant of sortilin, or a sortilin mimetic as defined herein, in the manufacture of a medicament for treating or preventing diabetes mellitus, insulin resistance, obesity, metabolic disorders, polycystic ovary syndrome, non-alcoholic fatty liver disease, retinopathy, neuropathy, nephropathy, Alzheimer's disease and/or cardiovascular disease in a subject in need thereof, wherein the sortilin mimetic is not SorCS1, SorCS2, SorCS3 or SorLA.

The invention further provides a method of treating or preventing a condition selected from diabetes mellitus, insulin resistance, obesity, metabolic disorders, polycystic ovary syndrome, non-alcoholic fatty liver disease, retinopathy, neuropathy, nephropathy, Alzheimer's disease and/or cardiovascular disease in a subject in need thereof, the method comprising administering a composition comprising a fragment, derivative or variant of sortilin, or a sortilin mimetic as defined herein to the subject.

The present invention also includes a method of manufacturing polypeptides or fragments thereof according to the invention comprising the step of expressing the nucleic acid of the invention in a host cell or other suitable expression system as indicated herein.

In order that the invention may be more clearly understood, embodiments thereof will now be described by way of example.

### Examples

### Example 1:

The percentage level of identity between hSORT1 and hSORCS1 were investigated by aligning the protein sequences. Alignment software used by the inventors can be accessed via Uniprot.org.

The data shows that between the human and murine forms of SORT1, there is a high level of homology (87 %). However, when comparing the homology within the Vps10p domain between hSORT1 and hSORCS1, it was found to be substantially lower (25 %) (Figure 1).

### Example 2:

Sortilin and insulin receptor binding was investigated using surface plasmon resonance analysis.

Soluble sortilin in concentrations ranging from 50-500 nM was assayed for binding to immobilized human insulin receptor (0.0609 pmol/mm²) and murine insulin receptor (0.0607 pmol/mm²) (Figure 2A and B). Sortilin showed dose-dependent binding to both the human and murine insulin receptor, with a Kd of approximately 40 nM. Insulin did not affect the binding between sortilin and the insulin receptor (Figure 2C), indicating the formation of a trimeric receptor complex. Co-immunoprecipitation confirmed a physical interaction between sortilin and the human receptor with DSP crosslinker.

The data shows that sortilin and the insulin receptor are able to physically interact in a cell free system (Biacore). The interaction between sortilin and the insulin receptor does not impact on the binding of insulin to the insulin receptor.

### Example 3:

RAP-purified soluble sortilin was tested in diabetic animal models (Db/Db) to investigate effects on plasma glucose levels, in steady state (basal fasting plasma glucose) and in dynamic state (IPGTT).

18 mice were ordered, aged 5 weeks at the point of delivery. The animals were allowed to acclimatise for 3 weeks prior to performing the experimental work. The animals were test fasted for 4 hrs and blood sampled 5 days before the experiment took place. The animals were randomised to either treatment or sham groups based on weight and plasma glucose levels. Following randomisation, the 18 db/db mice, now aged 8 weeks, were injected IP with 100 µg RAP-purified soluble sortilin (n=8) or saline (n=10) at 22:00 and at 08:00 the following morning. All animals were fasted from 22:00. The animals were subjected to an IPGTT at 09:00, with the dose of 1 mg/g BW in 200 mg/ml glucose (40 g = 200 µL) (Figure 3). The IPGTT curve showed a significant lower basal plasma glucose, and lower IPGTT AUC, both with and without correction for baseline plasma glucose (t=0 min).

### Example 4:

The RAP-purified construct has a C-terminal HIS-tag which may engage with the heparin-sulfate proteoglycans to inhibit its activity. Therefore, this interaction and the effects on plasma glucose levels were investigated.

20 mice were ordered, aged 5 weeks at delivery. The animals were allowed to acclimatise for 2 weeks prior to performing the experimental work. The animals were test fasted for 4 hrs and sampled 5 days before the experiment took place. The animals were then randomised to either treatment or sham groups based on weight (approximately 30 g) and plasma glucose levels. The 20 db/db mice, now aged 7 weeks, were injected IP with 100 µg RAP-purified soluble sortilin and heparin (200 U/kg) (n=7) or without heparin (n=6), or saline (n=7) at 22:00 and at 08:00 the following morning. All animals were fasted from 22:00. The animals were subjected to an IPGTT at 09:00, with a dose of 1 mg/g BW in 200 mg/ml glucose (40 g = 200 ul) (Figure 4). The IPGTT curve showed a trend towards a lower basal plasma glucose, and lower IPGTT AUC, both total and baseline corrected. There was an effect of RAP-purified sortilin and no effect of added heparin. Insulin response after glucose challenge was improved when animals were treated with soluble sortilin (+/- heparin).

HOMA1-IR (resistance index) is calculated as (FPI x FPG)/405 =HOMA1-IR, where FPI is fasting plasma insulin levels (mU/L), FPG is fasting plasma glucose (mg/dL) (Mather K. Am J Physiol Endocrinol Metab 296: E398-E399, 2009). QUICKI (sensitivity) Index is calculated as 1/(log(FPI mU/L)+log(FPG mg/dL) (Katz et al. JCEM 2000). Both QUICKI and HOMA1-IR indicates lower insulin resistance.

The data shows that RAP-purified soluble sortilin is active regardless of the presence of heparin during the glucose tolerance test.

### Sequences referenced throughout the specification and forming part of the description.

SEQ ID NO: 1 (VPS10p Domain- isoform 1) 
1   RGGRWRRSAP GEDEECGRVR DFVAKLANNT HQHVFDDLRG SVSLSWVGDS
51  TGVILVLTTF HVPLVIMTFG QSKLYRSEDY GKNFKDITDL INNTFIRTEF
101 GMAIGPENSG KVVLTAEVSG GSRGGRIFRS SDFAKNFVQT DLPFHPLTQM
151 MYSPQNSDYL LALSTENGLW VSKNFGGKWE EIHKAVCLAK WGSDNTIFFT
201 TYANGSCKAD LGALELWRTS DLGKSFKTIG VKIYSFGLGG RFLFASVMAD
251 KDTTRRIHVS TDQGDTWSMA QLPSVGQEQF YSILAANDDM VFMHVDEPGD
301 TGFGTIFTSD DRGIVYSKSL DRHLYTTTGG ETDFTNVTSL RGVYITSVLS
351 EDNSIQTMIT FDQGGRWTHL RKPENSECDA TAKNKNECSL HIHASYSISQ
401 KLNVPMAPLS  EPNAVGIVIA HGSVGDAISV MVPDVYISDD GGYSWTKMLE
451 GPHYYTILDS GGIIVAIEHS SRPINVIKFS TDEGQCWQTY TFTRDPIYFT
501 GLASEPGARS MNISIWGFTE SFLTSQWVSY TIDFKDILER
SEQ ID NO: 2 (VPS10p Domain + 10CCa Domain- isoform 1) 
1   RGGRWRRSAP GEDEECGRVR DFVAKLANNT HQHVFDDLRG SVSLSWVGDS
51  TGVILVLTTF HVPLVIMTFG QSKLYRSEDY GKNFKDITDL INNTFIRTEF
101 GMAIGPENSG KVVLTAEVSG GSRGGRIFRS SDFAKNFVQT DLPFHPLTQM
151 MYSPQNSDYL LALSTENGLW VSKNFGGKWE EIHKAVCLAK WGSDNTIFFT
201 TYANGSCKAD LGALELWRTS DLGKSFKTIG VKIYSFGLGG RFLFASVMAD
251 KDTTRRIHVS TDQGDTWSMA QLPSVGQEQF YSILAANDDM VFMHVDEPGD
301 TGFGTIFTSD DRGIVYSKSL DRHLYTTTGG ETDFTNVTSL RGVYITSVLS
351 EDNSIQTMIT FDQGGRWTHL RKPENSECDA TAKNKNECSL HIHASYSISQ
401 KLNVPMAPLS  EPNAVGIVIA HGSVGDAISV MVPDVYISDD GGYSWTKMLE
451 GPHYYTILDS GGIIVAIEHS SRPINVIKFS TDEGQCWQTY TFTRDPIYFT
501 GLASEPGARS MNISIWGFTE SFLTSQWVSY TIDFKDILER NCEEKDYTIW
551 LAHSTDPEDY EDGCILGYKE QFLRLRKSSM CQNGRDYVVT KQPSICL
SEQ ID NO: 3 (VPS10p Domain + 10CCb Domain- isoform 1) 
1   RGGRWRRSAP GEDEECGRVR DFVAKLANNT HQHVFDDLRG SVSLSWVGDS
51  TGVILVLTTF HVPLVIMTFG QSKLYRSEDY GKNFKDITDL INNTFIRTEF
101 GMAIGPENSG KVVLTAEVSG GSRGGRIFRS SDFAKNFVQT DLPFHPLTQM
151 MYSPQNSDYL LALSTENGLW VSKNFGGKWE EIHKAVCLAK WGSDNTIFFT
201 TYANGSCKAD LGALELWRTS DLGKSFKTIG VKIYSFGLGG RFLFASVMAD
251 KDTTRRIHVS TDQGDTWSMA QLPSVGQEQF YSILAANDDM VFMHVDEPGD
301 TGFGTIFTSD DRGIVYSKSL DRHLYTTTGG ETDFTNVTSL RGVYITSVLS
351 EDNSIQTMIT FDQGGRWTHL RKPENSECDA TAKNKNECSL HIHASYSISQ
401 KLNVPMAPLS  EPNAVGIVIA HGSVGDAISV MVPDVYISDD GGYSWTKMLE
451 GPHYYTILDS GGIIVAIEHS SRPINVIKFS TDEGQCWQTY TFTRDPIYFT
501 GLASEPGARS MNISIWGFTE SFLTSQWVSY TIDFKDILER CSLEDFLCDF
551 GYYRPENDSK CVEQPELKGH DLEFCLYGRE EHLTTNGYRK IPGDKCQGGV
601 NPVREVKDLK KKCTSNFLSP EKQ
SEQ ID NO: 4 (VPS10p Domain + 10CCa Domain + 10CCb Domain- isoform 1) 
1   RGGRWRRSAP GEDEECGRVR DFVAKLANNT HQHVFDDLRG SVSLSWVGDS
51  TGVILVLTTF HVPLVIMTFG QSKLYRSEDY GKNFKDITDL INNTFIRTEF
101 GMAIGPENSG KVVLTAEVSG GSRGGRIFRS SDFAKNFVQT DLPFHPLTQM
151 MYSPQNSDYL LALSTENGLW VSKNFGGKWE EIHKAVCLAK WGSDNTIFFT
201 TYANGSCKAD LGALELWRTS DLGKSFKTIG VKIYSFGLGG RFLFASVMAD
251 KDTTRRIHVS TDQGDTWSMA QLPSVGQEQF YSILAANDDM VFMHVDEPGD
 
301 TGFGTIFTSD DRGIVYSKSL DRHLYTTTGG ETDFTNVTSL RGVYITSVLS
351 EDNSIQTMIT FDQGGRWTHL RKPENSECDA TAKNKNECSL HIHASYSISQ
401 KLNVPMAPLS  EPNAVGIVIA HGSVGDAISV MVPDVYISDD GGYSWTKMLE
451 GPHYYTILDS GGIIVAIEHS SRPINVIKFS TDEGQCWQTY TFTRDPIYFT
501 GLASEPGARS MNISIWGFTE SFLTSQWVSY TIDFKDILER NCEEKDYTIW
551 LAHSTDPEDY EDGCILGYKE QFLRLRKSSM CQNGRDYVVT KQPSICLCSL
601 EDFLCDFGYY RPENDSKCVE QPELKGHDLE FCLYGREEHL TTNGYRKIPG
651 DKCQGGVNPV REVKDLKKKC TSNFLSPEKQ
SEQ ID NO: 5 (10CCa Domain- isoform 1) 
1 NCEEKDYTIW LAHSTDPEDY EDGCILGYKE QFLRLRKSSM CQNGRDYVVT
51 KQPSICL
SEQ ID NO: 6 (10CCb Domain- isoform 1) 
1 CSLEDFLCDF GYYRPENDSK CVEQPELKGH DLEFCLYGRE EHLTTNGYRK
51 IPGDKCQGGV NPVREVKDLK KKCTSNFLSP EKQ
SEQ ID NO: 7 (10CCa + 10CCb Domain- isoform 1) 
1 NCEEKDYTIW LAHSTDPEDY EDGCILGYKE QFLRLRKSSM CQNGRDYVVT
51 KQPSICLCSL EDFLCDFGYY RPENDSKCVE QPELKGHDLE FCLYGREEHL
101 TTNGYRKIPG DKCQGGVNPV REVKDLKKKC TSNFLSPEKQ
SEQ ID NO: 8 (IST218 after furin site plus HIS tag- isoform 1) 
1 SAPGEDEECG RVRDFVAKLA NNTHQHVFDD LRGSVSLSWV GDSTGVILVL
51 TTFHVPLVIM TFGQSKLYRS EDYGKNFKDI TDLINNTFIR TEFGMAIGPE
101 NSGKVVLTAE VSGGSRGGRI FRSSDFAKNF VQTDLPFHPL TQMMYSPQNS
151 DYLLALSTEN GLWVSKNFGG KWEEIHKAVC LAKWGSDNTI FFTTYANGSC
201 KADLGALELW RTSDLGKSFK TIGVKIYSFG LGGRFLFASV MADKDTTRRI
251 HVSTDQGDTW SMAQLPSVGQ EQFYSILAAN DDMVFMHVDE PGDTGFGTIF
301 TSDDRGIVYS KSLDRHLYTT TGGETDFTNV TSLRGVYITS VLSEDNSIQT
351 MITFDQGGRW THLRKPENSE CDATAKNKNE CSLHIHASYS ISQKLNVPMA
401 PLSEPNAVGI VIAHGSVGDA ISVMVPDVYI SDDGGYSWTK MLEGPHYYTI
451 LDSGGIIVAI EHSSRPINVI KFSTDEGQCW QTYTFTRDPI YFTGLASEPG
501 ARSMNISIWG FTESFLTSQW VSYTIDFKDI LERNCEEKDY TIWLAHSTDP
551 EDYEDGCILG YKEQFLRLRK SSMCQNGRDY VVTKQPSICL CSLEDFLCDF
601 GYYRPENDSK CVEQPELKGH DLEFCLYGRE EHLTTNGYRK IPGDKCQGGV
651 NPVREVKDLK KKCTSNFLSP EKQNSKSNSH HHHHH
SEQ ID NO: 9 (IST218 after furin site minus HIS tag- isoform 1) 
1 SAPGEDEECG RVRDFVAKLA NNTHQHVFDD LRGSVSLSWV GDSTGVILVL
51 TTFHVPLVIM TFGQSKLYRS EDYGKNFKDI TDLINNTFIR TEFGMAIGPE
101 NSGKVVLTAE VSGGSRGGRI FRSSDFAKNF VQTDLPFHPL TQMMYSPQNS
151 DYLLALSTEN GLWVSKNFGG KWEEIHKAVC LAKWGSDNTI FFTTYANGSC
201 KADLGALELW RTSDLGKSFK TIGVKIYSFG LGGRFLFASV MADKDTTRRI
251 HVSTDQGDTW SMAQLPSVGQ EQFYSILAAN DDMVFMHVDE PGDTGFGTIF
301 TSDDRGIVYS KSLDRHLYTT TGGETDFTNV TSLRGVYITS VLSEDNSIQT
351 MITFDQGGRW THLRKPENSE CDATAKNKNE CSLHIHASYS ISQKLNVPMA
401 PLSEPNAVGI VIAHGSVGDA ISVMVPDVYI SDDGGYSWTK MLEGPHYYTI
451 LDSGGIIVAI EHSSRPINVI KFSTDEGQCW QTYTFTRDPI YFTGLASEPG
501 ARSMNISIWG FTESFLTSQW VSYTIDFKDI LERNCEEKDY TIWLAHSTDP
551 EDYEDGCILG YKEQFLRLRK SSMCQNGRDY VVTKQPSICL CSLEDFLCDF
601 GYYRPENDSK CVEQPELKGH DLEFCLYGRE EHLTTNGYRK IPGDKCQGGV
 651 NPVREVKDLK KKCTSNFLSP EKQNSKSNS
SEQ ID NO: 10 (SKT1 after furin site plus HIS tag + TEV site- isoform 1) 
1 SAPGHHHHHH HENLYFQSED EECGRVRDFV AKLANNTHQH VFDDLRGSVS
51 LSWVGDSTGV ILVLTTFHVP LVIMTFGQSK LYRSEDYGKN FKDITDLINN
101 TFIRTEFGMA IGPENSGKVV LTAEVSGGSR GGRIFRSSDF AKNFVQTDLP
151 FHPLTQMMYS PQNSDYLLAL STENGLWVSK NFGGKWEEIH KAVCLAKWGS
201 DNTIFFTTYA NGSCKADLGA LELWRTSDLG KSFKTIGVKI YSFGLGGRFL
251 FASVMADKDT TRRIHVSTDQ GDTWSMAQLP SVGQEQFYSI LAANDDMVFM
301 HVDEPGDTGF GTIFTSDDRG IVYSKSLDRH LYTTTGGETD FTNVTSLRGV
351 YITSVLSEDN SIQTMITFDQ GGRWTHLRKP ENSECDATAK NKNECSLHIH
401 ASYSISQKLN VPMAPLSEPN AVGIVIAHGS VGDAISVMVP DVYISDDGGY
451 SWTKMLEGPH YYTILDSGGI IVAIEHSSRP INVIKFSTDE GQCWQTYTFT
501 RDPIYFTGLA SEPGARSMNI SIWGFTESFL TSQWVSYTID FKDILERNCE
551 EKDYTIWLAH STDPEDYEDG CILGYKEQFL RLRKSSMCQN GRDYVVTKQP
601 SICLCSLEDF LCDFGYYRPE NDSKCVEQPE LKGHDLEFCL YGREEHLTTN
651 GYRKIPGDKC QGGVNPVREV KDLKKKCTSN
SEQ ID NO: 11 (SKT1 after furin site minus HIS tag + TEV site- isoform 1) 
1 EDEECGRVRD FVAKLANNTH QHVFDDLRGS VSLSWVGDST GVILVLTTFH
51 VPLVIMTFGQ SKLYRSEDYG KNFKDITDLI NNTFIRTEFG MAIGPENSGK
101 VVLTAEVSGG SRGGRIFRSS DFAKNFVQTD LPFHPLTQMM YSPQNSDYLL
151 ALSTENGLWV SKNFGGKWEE IHKAVCLAKW GSDNTIFFTT YANGSCKADL
201 GALELWRTSD LGKSFKTIGV KIYSFGLGGR FLFASVMADK DTTRRIHVST
251 DQGDTWSMAQ LPSVGQEQFY SILAANDDMV FMHVDEPGDT GFGTIFTSDD
301 RGIVYSKSLD RHLYTTTGGE TDFTNVTSLR GVYITSVLSE DNSIQTMITF
351 DQGGRWTHLR KPENSECDAT AKNKNECSLH IHASYSISQK LNVPMAPLSE
401 PNAVGIVIAH GSVGDAISVM VPDVYISDDG GYSWTKMLEG PHYYTILDSG
451 GIIVAIEHSS RPINVIKFST DEGQCWQTYT FTRDPIYFTG LASEPGARSM
501 NISIWGFTES FLTSQWVSYT IDFKDILERN CEEKDYTIWL AHSTDPEDYE
551 DGCILGYKEQ FLRLRKSSMC QNGRDYVVTK QPSICLCSLE DFLCDFGYYR
601 PENDSKCVEQ PELKGHDLEF CLYGREEHLT TNGYRKIPGD KCQGGVNPVR
651 EVKDLKKKCT SN
SEQ ID NO: 12 (full length sortilin- isoform 1) 
1 
51 
101 
151 
201 
251 
301 
351 
401 
451 
501 
551 
601 
651 
701 
751 
801 
SEQ ID NO: 13 (full length sortilin- isoform 2) 
1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCTDL GALELWRTSD LGKSFKTIGV
301 KIYSFGLGGR FLFASVMADK DTTRRIHVST DQGDTWSMAQ LPSVGQEQFY
351 SILAANDDMV FMHVDEPGDT GFGTIFTSDD RGIVYSKSLD RHLYTTTGGE
401 TDFTNVTSLR GVYITSVLSE DNSIQTMITF DQGGRWTHLR KPENSECDAT
451 AKNKNECSLH IHASYSISQK LNVPMAPLSE PNAVGIVIAH GSVGDAISVM
501 VPDVYISDDG GYSWTKMLEG PHYYTILDSG GIIVAIEHSS RPINVIKFST
551 DEGQCWQTYT FTRDPIYFTG LASEPGARSM NISIWGFTES FLTSQWVSYT
601 IDFKDILERN CEEKDYTIWL AHSTDPEDYE DGCILGYKEQ FLRLRKSSVC
651 QNGRDYVVTK QPSICLCSLE DFLCDFGYYR PENDSKCVEQ PELKGHDLEF
701 CLYGREEHLT TNGYRKIPGD KCQGGVNPVR EVKDLKKKCT SNFLSPEKQN
751 SKSNSVPIIL AIVGLMLVTV VAGVLIVKKY VCGGRFLVHR YSVLQQHAEA
801 NGVDGVDALD TASHTNKSGY HDDSDEDLLE
SEQ ID NO: 14 (VPS10p Domain- isoform 2) 
1 RGGRWRRSAP GEDEECGRVR DFVAKLANNT HQHVFDDLRG SVSLSWVGDS
51 TGVILVLTTF HVPLVIMTFG QSKLYRSEDY GKNFKDITDL INNTFIRTEF
101 GMAIGPENSG KVVLTAEVSG GSRGGRIFRS SDFAKNFVQT DLPFHPLTQM
151 MYSPQNSDYL LALSTENGLW VSKNFGGKWE EIHKAVCLAK WGSDNTIFFT
201 TYANGSCTDL GALELWRTSD LGKSFKTIGV KIYSFGLGGR FLFASVMADK
251 DTTRRIHVST DQGDTWSMAQ LPSVGQEQFY SILAANDDMV FMHVDEPGDT
300 GFGTIFTSDD RGIVYSKSLD RHLYTTTGGE TDFTNVTSLR GVYITSVLSE
351 DNSIQTMITF DQGGRWTHLR KPENSECDAT AKNKNECSLH IHASYSISQK
400 LNVPMAPLSE PNAVGIVIAH GSVGDAISVM VPDVYISDDG GYSWTKMLEG
401 PHYYTILDSG GIIVAIEHSS RPINVIKFST DEGQCWQTYT FTRDPIYFTG
451 LASEPGARSM NISIWGFTES FLTSQWVSYT IDFKDILER
SEQ ID NO: 15 (VPS10p Domain + 10CCa Domain- isoform 2) 
1 RGGRWRRSAP GEDEECGRVR DFVAKLANNT HQHVFDDLRG SVSLSWVGDS
51 TGVILVLTTF HVPLVIMTFG QSKLYRSEDY GKNFKDITDL INNTFIRTEF
101 GMAIGPENSG KVVLTAEVSG GSRGGRIFRS SDFAKNFVQT DLPFHPLTQM
151 MYSPQNSDYL LALSTENGLW VSKNFGGKWE EIHKAVCLAK WGSDNTIFFT
201 TYANGSCTDL GALELWRTSD LGKSFKTIGV KIYSFGLGGR FLFASVMADK
251 DTTRRIHVST DQGDTWSMAQ LPSVGQEQFY SILAANDDMV FMHVDEPGDT
300 GFGTIFTSDD RGIVYSKSLD RHLYTTTGGE TDFTNVTSLR GVYITSVLSE
351 DNSIQTMITF DQGGRWTHLR KPENSECDAT AKNKNECSLH IHASYSISQK
400 LNVPMAPLSE PNAVGIVIAH GSVGDAISVM VPDVYISDDG GYSWTKMLEG
401 PHYYTILDSG GIIVAIEHSS RPINVIKFST DEGQCWQTYT FTRDPIYFTG
451 LASEPGARSM NISIWGFTES FLTSQWVSYT IDFKDILERN CEEKDYTIWL
501 AHSTDPEDYE DGCILGYKEQ FLRLRKSSVC QNGRDYVVTK QPSICL
SEQ ID NO: 16 (VPS10p Domain + 10CCb Domain- isoform 2) 
1 RGGRWRRSAP GEDEECGRVR DFVAKLANNT HQHVFDDLRG SVSLSWVGDS
51 TGVILVLTTF HVPLVIMTFG QSKLYRSEDY GKNFKDITDL INNTFIRTEF
101 GMAIGPENSG KVVLTAEVSG GSRGGRIFRS SDFAKNFVQT DLPFHPLTQM
151 MYSPQNSDYL LALSTENGLW VSKNFGGKWE EIHKAVCLAK WGSDNTIFFT
201 TYANGSCTDL GALELWRTSD LGKSFKTIGV KIYSFGLGGR FLFASVMADK
 
251 DTTRRIHVST DQGDTWSMAQ LPSVGQEQFY SILAANDDMV FMHVDEPGDT
300 GFGTIFTSDD RGIVYSKSLD RHLYTTTGGE TDFTNVTSLR GVYITSVLSE
351 DNSIQTMITF DQGGRWTHLR KPENSECDAT AKNKNECSLH IHASYSISQK
400 LNVPMAPLSE PNAVGIVIAH GSVGDAISVM VPDVYISDDG GYSWTKMLEG
401 PHYYTILDSG GIIVAIEHSS RPINVIKFST DEGQCWQTYT FTRDPIYFTG
451 LASEPGARSM NISIWGFTES FLTSQWVSYT IDFKDILERC SLEDFLCDFG
501 YYRPENDSKC VEQPELKGHD LEFCLYGREE HLTTNGYRKI PGDKCQGGVN
551 PVREVKDLKK KCTSNFLSPE KQ
SEQ ID NO: 17 (VPS10p Domain + 10CCa Domain + 10CCb Domain- isoform 2) 
1   RGGRWRRSAP GEDEECGRVR DFVAKLANNT HQHVFDDLRG SVSLSWVGDS
51   TGVILVLTTF HVPLVIMTFG QSKLYRSEDY GKNFKDITDL INNTFIRTEF
101 GMAIGPENSG KVVLTAEVSG GSRGGRIFRS SDFAKNFVQT DLPFHPLTQM
151 MYSPQNSDYL LALSTENGLW VSKNFGGKWE EIHKAVCLAK WGSDNTIFFT
201 TYANGSCTDL GALELWRTSD LGKSFKTIGV KIYSFGLGGR FLFASVMADK
251 DTTRRIHVST DQGDTWSMAQ LPSVGQEQFY SILAANDDMV FMHVDEPGDT
300 GFGTIFTSDD RGIVYSKSLD RHLYTTTGGE TDFTNVTSLR GVYITSVLSE
351 DNSIQTMITF DQGGRWTHLR KPENSECDAT AKNKNECSLH IHASYSISQK
400 LNVPMAPLSE PNAVGIVIAH GSVGDAISVM VPDVYISDDG GYSWTKMLEG
401 PHYYTILDSG GIIVAIEHSS RPINVIKFST DEGQCWQTYT FTRDPIYFTG
451 LASEPGARSM NISIWGFTES FLTSQWVSYT IDFKDILERN CEEKDYTIWL
501 AHSTDPEDYE DGCILGYKEQ FLRLRKSSVC QNGRDYVVTK QPSICLCSLE
551 DFLCDFGYYR PENDSKCVEQ PELKGHDLEF CLYGREEHLT TNGYRKIPGD
601 KCQGGVNPVR EVKDLKKKCT SNFLSPEKQ
SEQ ID NO: 18 (10CCa Domain- isoform 2) 
1 NCEEKDYTIW LAHSTDPEDY EDGCILGYKE QFLRLRKSSV CQNGRDYVVT
51 KQPSICL
SEQ ID NO: 19 (10CCa Domain + 10CCb Domain- isoform 2) 
1 NCEEKDYTIW LAHSTDPEDY EDGCILGYKE QFLRLRKSSV CQNGRDYVVT
51 KQPSICLCSL EDFLCDFGYY RPENDSKCVE QPELKGHDLE FCLYGREEHL
101 TTNGYRKIPG DKCQGGVNPV REVKDLKKKC TSNFLSPEKQ
SEQ ID NO: 20 (IST218 after furin site plus HIS tag- isoform 2) 
1 SAPGEDEECG RVRDFVAKLA NNTHQHVFDD LRGSVSLSWV GDSTGVILVL
51 TTFHVPLVIM TFGQSKLYRS EDYGKNFKDI TDLINNTFIR TEFGMAIGPE
101 NSGKVVLTAE VSGGSRGGRI FRSSDFAKNF VQTDLPFHPL TQMMYSPQNS
151 DYLLALSTEN GLWVSKNFGG KWEEIHKAVC LAKWGSDNTI FFTTYANGSC
201 TDLGALELWR TSDLGKSFKT IGVKIYSFGL GGRFLFASVM ADKDTTRRIH
251 VSTDQGDTWS MAQLPSVGQE QFYSILAAND DMVFMHVDEP GDTGFGTIFT
301 SDDRGIVYSK SLDRHLYTTT GGETDFTNVT SLRGVYITSV LSEDNSIQTM
351 ITFDQGGRWT HLRKPENSEC DATAKNKNEC SLHIHASYSI SQKLNVPMAP
401 LSEPNAVGIV IAHGSVGDAI SVMVPDVYIS DDGGYSWTKM LEGPHYYTIL
451 DSGGIIVAIE HSSRPINVIK FSTDEGQCWQ TYTFTRDPIY FTGLASEPGA
501 RSMNISIWGF TESFLTSQWV SYTIDFKDIL ERNCEEKDYT IWLAHSTDPE
551 DYEDGCILGY KEQFLRLRKS SVCQNGRDYV VTKQPSICLC SLEDFLCDFG
601 YYRPENDSKC VEQPELKGHD LEFCLYGREE HLTTNGYRKI PGDKCQGGVN
651 PVREVKDLKK KCTSNFLSPE KQNSKSNSHH HHHH
SEQ ID NO: 21 (IST218 after furin site minus HIS tag- isoform 2) 
1 SAPGEDEECG RVRDFVAKLA NNTHQHVFDD LRGSVSLSWV GDSTGVILVL
51 TTFHVPLVIM TFGQSKLYRS EDYGKNFKDI TDLINNTFIR TEFGMAIGPE
101 NSGKVVLTAE VSGGSRGGRI FRSSDFAKNF VQTDLPFHPL TQMMYSPQNS
151 DYLLALSTEN GLWVSKNFGG KWEEIHKAVC LAKWGSDNTI FFTTYANGSC
201 TDLGALELWR TSDLGKSFKT IGVKIYSFGL GGRFLFASVM ADKDTTRRIH
251 VSTDQGDTWS MAQLPSVGQE QFYSILAAND DMVFMHVDEP GDTGFGTIFT
301 SDDRGIVYSK SLDRHLYTTT GGETDFTNVT SLRGVYITSV LSEDNSIQTM
351 ITFDQGGRWT HLRKPENSEC DATAKNKNEC SLHIHASYSI SQKLNVPMAP
401 LSEPNAVGIV IAHGSVGDAI SVMVPDVYIS DDGGYSWTKM LEGPHYYTIL
451 DSGGIIVAIE HSSRPINVIK FSTDEGQCWQ TYTFTRDPIY FTGLASEPGA
501 RSMNISIWGF TESFLTSQWV SYTIDFKDIL ERNCEEKDYT IWLAHSTDPE
551 DYEDGCILGY KEQFLRLRKS SVCQNGRDYV VTKQPSICLC SLEDFLCDFG
601 YYRPENDSKC VEQPELKGHD LEFCLYGREE HLTTNGYRKI PGDKCQGGVN
651 PVREVKDLKK KCTSNFLSPE KQNSKSNS
SEQ ID NO: 22 (SKT1 after furin site plus HIS tag + TEV site- isoform 2) 
1 SAPGHHHHHH HENLYFQSED EECGRVRDFV AKLANNTHQH VFDDLRGSVS
51 LSWVGDSTGV ILVLTTFHVP LVIMTFGQSK LYRSEDYGKN FKDITDLINN
101 TFIRTEFGMA IGPENSGKVV LTAEVSGGSR GGRIFRSSDF AKNFVQTDLP
151 FHPLTQMMYS PQNSDYLLAL STENGLWVSK NFGGKWEEIH KAVCLAKWGS
201 DNTIFFTTYA NGSCTDLGAL ELWRTSDLGK SFKTIGVKIY SFGLGGRFLF
251 ASVMADKDTT RRIHVSTDQG DTWSMAQLPS VGQEQFYSIL AANDDMVFMH
301 VDEPGDTGFG TIFTSDDRGI VYSKSLDRHL YTTTGGETDF TNVTSLRGVY
351 ITSVLSEDNS IQTMITFDQG GRWTHLRKPE NSECDATAKN KNECSLHIHA
401 SYSISQKLNV PMAPLSEPNA VGIVIAHGSV GDAISVMVPD VYISDDGGYS
451 WTKMLEGPHY YTILDSGGII VAIEHSSRPI NVIKFSTDEG QCWQTYTFTR
501 DPIYFTGLAS EPGARSMNIS IWGFTESFLT SQWVSYTIDF KDILERNCEE
551 KDYTIWLAHS TDPEDYEDGC ILGYKEQFLR LRKSSVCQNG RDYVVTKQPS
601 ICLCSLEDFL CDFGYYRPEN DSKCVEQPEL KGHDLEFCLY GREEHLTTNG
651 YRKIPGDKCQ GGVNPVREVK DLKKKCTSN
SEQ ID NO: 23 (SKT1 after furin site minus HIS tag + TEV site- isoform 2) 
1   EDEECGRVRD FVAKLANNTH QHVFDDLRGS VSLSWVGDST GVILVLTTFH
51   VPLVIMTFGQ SKLYRSEDYG KNFKDITDLI NNTFIRTEFG MAIGPENSGK
101 VVLTAEVSGG SRGGRIFRSS DFAKNFVQTD LPFHPLTQMM YSPQNSDYLL
151 ALSTENGLWV SKNFGGKWEE IHKAVCLAKW GSDNTIFFTT YANGSCTDLG
201 ALELWRTSDL GKSFKTIGVK IYSFGLGGRF LFASVMADKD TTRRIHVSTD
251 QGDTWSMAQL PSVGQEQFYS ILAANDDMVF MHVDEPGDTG FGTIFTSDDR
301 GIVYSKSLDR HLYTTTGGET DFTNVTSLRG VYITSVLSED NSIQTMITFD
351 QGGRWTHLRK PENSECDATA KNKNECSLHI HASYSISQKL NVPMAPLSEP
401 NAVGIVIAHG SVGDAISVMV PDVYISDDGG YSWTKMLEGP HYYTILDSGG
451 IIVAIEHSSR PINVIKFSTD EGQCWQTYTF TRDPIYFTGL ASEPGARSMN
501 ISIWGFTESF LTSQWVSYTI DFKDILERNC EEKDYTIWLA HSTDPEDYED
551 GCILGYKEQF LRLRKSSVCQ NGRDYVVTKQ PSICLCSLED FLCDFGYYRP
601 ENDSKCVEQP ELKGHDLEFC LYGREEHLTT NGYRKIPGDK CQGGVNPVRE
651 VKDLKKKCTS N
SEQ ID NO: 24 (TEV cleavage site option 1)
   1 ENLYFQG
SEQ ID NO: 25 (TEV cleavage site option 2)
   1 ENLYFQS
SEQ ID NO: 26 (C terminal HIS tag)
   1 HHHHHH
SEQ ID NO: 27 (N terminal HIS tag)
   1 HHHHHHH

## Claims

1. A composition comprising a fragment, derivative or variant of sortilin, or a sortilin mimetic, for use in medicine, wherein the sortilin mimetic is not SorCS1, SorCS2, SorCS3 or SorLA.

2. The composition for use according to claim 1, wherein the composition is for use in treating or preventing diabetes mellitus, insulin resistance, obesity, metabolic disorders, polycystic ovary syndrome, non-alcoholic fatty liver disease, retinopathy, neuropathy, nephropathy, Alzheimer's disease and/or cardiovascular disease in a subject in need thereof.

3. The composition for use according to claim 1 or 2, wherein said sortilin mimetic is an antibody, nanobody or aptamer.

4. The composition for use according to claim 1 or 2, wherein said fragment, derivative or variant comprises a VPS10p, 10CCa or 10CCb domain.

5. The composition for use according to claim 1, 2 or 4, wherein said fragment, derivative or variant of sortilin comprises the amino acid sequence of SEQ ID NO: 1, SEQ ID NO: 2, SEQ ID NO: 3, SEQ ID NO: 4, SEQ ID NO: 5, SEQ ID NO: 6, SED ID NO: 7, SEQ ID NO: 8, SEQ ID NO: 9, SEQ ID NO: 10, SEQ ID NO: 11, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22 or SEQ ID NO: 23, or an amino acid sequence having greater than 70 % homology to said amino acid sequence.

6. The composition for use according to any of claims 1 to 5, wherein said fragment, derivative or variant of sortilin, or sortilin mimetic, comprises an additional moiety and/or a modification, optionally, wherein the additional moiety is a linker molecule and/or wherein the modification is PEGylation, glycosylation or acetylation.

7. The composition for use according to any of claims 1 to 6, wherein the fragment, derivative or variant of sortilin, or sortilin mimetic binds to an insulin receptor, preferably a human and/or murine insulin receptor.

8. An isolated polypeptide comprising the amino acid sequence of SEQ ID NO: 1, SEQ ID NO: 2, SEQ ID NO: 3, SEQ ID NO: 4, SEQ ID NO: 5, SEQ ID NO: 6, SED ID NO: 7, SEQ ID NO: 8, SEQ ID NO: 9, SEQ ID NO: 10, SEQ ID NO: 11, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22 or SEQ ID NO: 23, or an amino acid sequence having greater than 70 % homology to said amino acid sequence, or a fragment thereof.

9. A nucleic acid encoding a polypeptide according to claim 8.

10. A nucleic acid that hybridises to a nucleic acid according to claim 9.

11. An expression vector comprising a nucleic acid according to claim 9 or 10.

12. A host cell or cell free expression system comprising an expression vector according to claim 11.

13. A pharmaceutical composition comprising an isolated polypeptide, nucleotide, vector or host cell according to any of claim 8, 9, 10, 11 or 12 and pharmaceutically acceptable carriers or excipients.

14. The pharmaceutical composition according to claim 13 for use in medicine.

15. The pharmaceutical composition for use according to claim 14, wherein the composition is for use in treating or preventing diabetes mellitus, insulin resistance, obesity, metabolic disorders polycystic ovary syndrome, non-alcoholic fatty liver disease, retinopathy, neuropathy, nephropathy, Alzheimer's disease and/or cardiovascular disease in a subject in need thereof.
